(19) Europäisches Patentamt
European Patent Office
Office européen des brevets

(11) **EP 3 325 624 B1**

(12) **EUROPEAN PATENT SPECIFICATION**

(45) Date of publication and mention
of the grant of the patent:
**31.03.2021 Bulletin 2021/13**

(21) Application number: **16831094.4**

(22) Date of filing: **21.07.2016**

(51) Int Cl.:
*C12N 15/11* (2006.01)          *C12N 15/113* (2010.01)
*C40B 40/06* (2006.01)          *C40B 40/08* (2006.01)
*C12Q 1/689* (2018.01)

(86) International application number:
**PCT/US2016/043295**

(87) International publication number:
**WO 2017/019440 (02.02.2017 Gazette 2017/05)**

(54) **METHODS FOR DETECTING AND TREATING LOW-VIRULENCE INFECTIONS**

VERFAHREN ZUR DETEKTION UND BEHANDLUNG VON INFEKTIONEN MIT NIEDRIGER VIRULENZ

PROCÉDÉS PERMETTANT DE DÉTECTER ET TRAITER DES INFECTIONS À FAIBLE VIRULENCE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.07.2015 US 201562196508 P**

(43) Date of publication of application:
**30.05.2018 Bulletin 2018/22**

(73) Proprietor: **ECM Diagnostics, Inc.
Aventura, FL 33180 (US)**

(72) Inventors:
• **CAPOOR, Manu
La Jolla, CA 92037 (US)**
• **SLABY, Ondrej
La Jolla, CA 92037 (US)**

(74) Representative: **Grund, Martin
Grund Intellectual Property Group
Patentanwalt und Solicitor PartG mbB
Postfach 44 05 16
80754 München (DE)**

(56) References cited:
**US-A1- 2007 065 844    US-A1- 2009 150 084
US-A1- 2013 225 440    US-A1- 2014 377 278**

• **ROLLASON JESS ET AL: "Genotypic and antimicrobial characterisation of Propionibacterium acnes isolates from surgically excised lumbar disc herniations.", BIOMED RESEARCH INTERNATIONAL 2013, vol. 2013, 2013, page 530382, XP002788174, ISSN: 2314-6141**
• **HANNE B. ALBERT ET AL: "Does nuclear tissue infected with bacteria following disc herniations lead to Modic changes in the adjacent vertebrae?", EUROPEAN SPINE JOURNAL, vol. 22, no. 4, 1 April 2013 (2013-04-01), pages 690-696, XP055163142, ISSN: 0940-6719, DOI: 10.1007/s00586-013-2674-z**
• **UÇKAY I ET AL: "Spondylodiscitis due to Propionibacterium acnes: report of twenty-nine cases and a review of the literature.", CLINICAL MICROBIOLOGY AND INFECTION : THE OFFICIAL PUBLICATION OF THE EUROPEAN SOCIETY OF CLINICAL MICROBIOLOGY AND INFECTIOUS DISEASES APR 2010, vol. 16, no. 4, April 2010 (2010-04), pages 353-358, XP55544588, ISSN: 1469-0691**
• **STIRLING A ET AL: "Association between sciatica and Propionibacterium acnes", LANCET, ELSEVIER, AMSTERDAM, NL, vol. 357, no. 9273, 23 June 2001 (2001-06-23), pages 2024-2025, XP004247724, ISSN: 0140-6736, DOI: 10.1016/S0140-6736(00)05109-6**

EP 3 325 624 B1

- CHRISTOF BIRKENMAIER: "Should We Start Treating Chronic Low Back Pain with Antibiotics Rather than with Pain Medications?", THE KOREAN JOURNAL OF PAIN, vol. 26, no. 4, 1 January 2013 (2013-01-01), page 327, XP55544380, ISSN: 2005-9159, DOI: 10.3344/kjp.2013.26.4.327
- ACHERMANN YVONNE ET AL: "Immunoproteomic Identification of In Vivo-Produced Propionibacterium acnes Proteins in a Rabbit Biofilm Infection Model", CLINICAL AND VACCINE IMMUNOLOGY, vol. 22, no. 5, May 2015 (2015-05), pages 467-476, XP55544907,
- ALBERT ET AL.: 'Does nuclear tissue infected with bacteria following disc herniations lead to Modic changes in the adjacent vertebrae?' EUR SPINE J. vol. 22, no. 4, 10 February 2013, pages 690 - 696, XP055163142
- HEIKKINEN ET AL.: 'Prediction of microRNA targets in Caenorhabditis elegans using a self-organizing map' BIOINFORMATICS vol. 27, no. 9, 21 March 2011, pages 1247 - 1254, XP055350530
- ALBERT ET AL.: 'Antibiotic treatment in patients with chronic low back pain and vertebral bone edema (Modic type 1 changes): a double-blind randomized clinical controlled trial of efficacy' EUR SPINE J. vol. 22, no. 4, 13 February 2013, pages 697 - 707, XP002725607

**Description**

BACKGROUND

The human body is a superorganism in which thousands of microbial species continually interact with the human body. As of March 2014, the Genomes Online database lists 2,723 completed and published bacterial genomes detected in the human body with at least 14,867 in progress. Studies have revealed the presence of thousands of previously unknown microbes in human tissue and blood. In fact, polybacterial and chronic pathogens have even been detected in environments that were previously thought to be sterile. As a consequence, a range of physical and neurological inflammatory diseases are now thought to be associated with shifts in microbiome composition. For example, evidence suggests that commensal bacteria, such as *Propionibacterium acnes* (*P. acnes*), a normal inhabitant of the human skin, may be responsible for low-virulence infections, including low-virulence infections associated with chronic low back pain and intervertebral disc disease. As an example, Rollason et al. could show the presence of *P. acnes* in excised disc material from patients with a lumbar disc herniation by metagenomics analysis (Rollason et al., "Genotypic antimicrobial characterization of Propionibacterium acnes isolates from surgically excised lumbar disc herniations", 2013).

Some pathogens, including commensal pathogens, can be detected in biological tissue by culture or molecular techniques such as PCR. However, the vast majority of the microorganisms cannot be cultured under standard conditions used for diagnostic purposes. Further, if the suspected pathogen is a commensal microorganism that is ubiquitous in certain host tissues, there will be an unacceptably high rate of false positives due to sample contamination, particularly when using molecular detection assays.

The present invention provides methods for identifying low-virulence infections with *Propionibacterium acnes* , including by distinguishing true infections from false positives, to thereby support responsible and cost-effective medical care. Further disclosed are methods for treating low-virulence infections, including infections associated with intervertebral disc disease or chronic low back pain. Other objectives of the present invention will be apparent from the following detailed description.

SUMMARY OF THE INVENTION

The invention is defined by the appended claims.

In one aspect, the invention provides methods for identifying low virulence infections with *Propionibacterium acnes,* by determining the presence of *Propionibacterium acnes* in a patient intravertebral disc sample, and by discriminating instances of chronic infection by *Propionibacterium acnes* from false positives (e.g., distinguish infection from contamination of the sample by commensal microflora). The invention in these embodiments is useful for the diagnosis of a low virulence infection, such as an infection of the intervertebral disc. For example, a sample that tests positive for the commensal microflora by one or more of microbiology culture, DNA analysis, immunochemistry, and microscopy, is evaluated for a host cell microRNA (miRNA) signature to rule out false positives (thereby confirming a low virulence infection). The surgeon or treating physician may then administer an appropriate treatment for patients identified as having a low-virulence infection.

In other aspects, the invention provides miRNA signatures that distinguish (with high sensitivity and specificity) samples that are positive for a low virulence infection with *Propionibacterium acnes* from samples that are negative for a low virulence infection. These tests can be combined with analysis from molecular assays, microbial culture, and sample microscopy, to discriminate false positives, or in some embodiments can be used independently to identify positive samples. An exemplary miRNA score for *P. acnes* infection can be determined by scoring the relative expression levels of miR-29a-3p and miR-574-3p. A diagnostic miRNA score (DMS) based on the following formula resulted in 95% accuracy in a validation study, with positive samples having a score of less than or equal to -0.01: DMS = 18.71 - 11.24 * log10 (miR-29a-3p) + 10.4 * log10 (miR-574-3p).

Other RNA signatures can be trained from RNA profiles of samples that are positive or abundant for *P. acnes* (or other commensal microorganism) and samples that test negative (or non-abundant) for *P. acnes.* For example, samples can be binned based on detection (or detection level) of the commensal microorganism by quantitative PCR and at least one other technique, such as microbial culture, immunochemistry, or spectroscopy.

In various embodiments the diagnostic test uses a profile of non-coding RNAs of the host cells in the sample, to discriminate low virulence infection from non-infected samples (and, in some embodiments, from false positives). miRNA profiles of host tissue are able to discriminate chronic low virulence infections, even though these low-grade infections cause only minor inflammatory responses on the local level.

The invention can be applied to surgical samples, to guide medical care post-surgery, or can be applied to biopsy samples, to guide treatment pre-surgery, and/or to potentially reduce the need for more invasive procedures by treating low virulence infections.

Further disclosed are methods for treating low virulence infections. The method may comprise treating a low-virulence infection with an antibiotic that is administered systemically, or with an antibiotic or antiseptic that is locally administered. Low virulence infections can be identified according to the methods described herein.

Other aspects and embodiments of the invention will be apparent from the following detailed description.

DESCRIPTION OF THE FIGURES

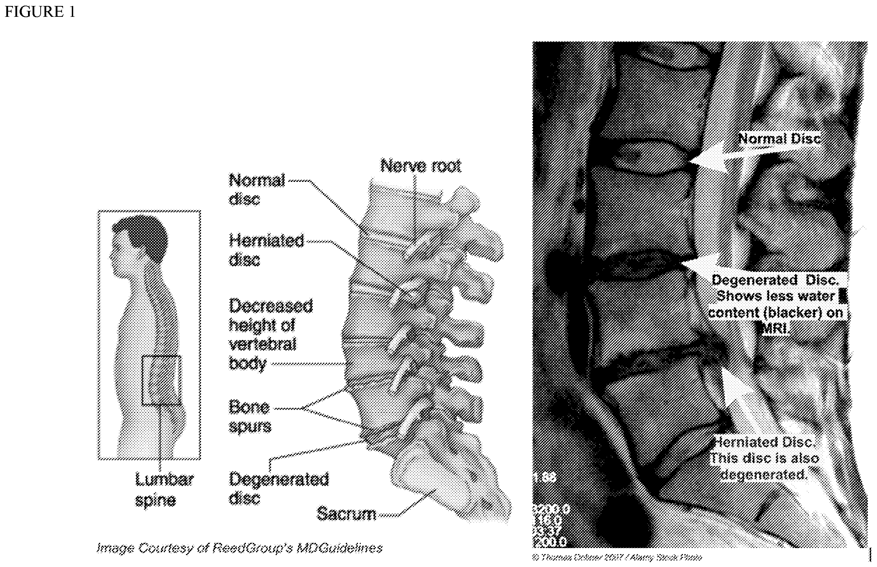

FIGURE 1 provides an illustration of Degenerative Disc Disease.

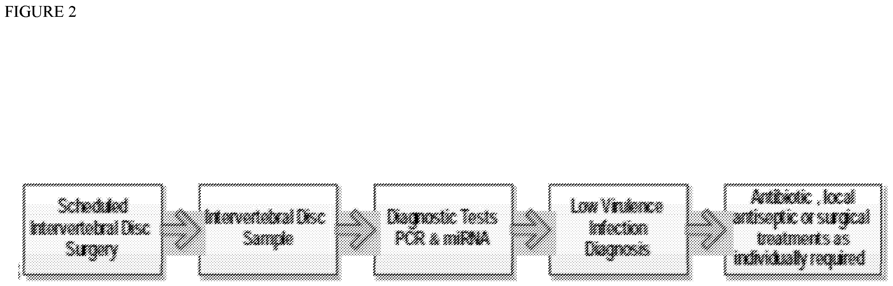

FIGURE 2 is a flow chart for embodiments of the invention, where patients are scheduled for Intervertebral Disc Surgery, and an Intervertebral Disc Sample is taken from the surgical site. Diagnostic tests (such as a PCR assay) are conducted on the sample to detect the presence or absence, or to quantify, commensal pathogens in the sample. Where the sample is positive for commensal pathogens, miRNA profiles are conducted to discriminate true infections from false positives (contamination). Where a low-virulent infection is confirmed, further treatment is recommended for the patient, such as oral, intravenous, or intervertebral antibiotic, local antiseptic treatment, or further surgical treatment.

FIGURE 3 is a schematic drawing of a human vertebra and intervertebral disc (IVD). (a) Transversal view of a lumbar vertebra. (b) IVD viewed from an anterior angle with partially removed AF lamellae. (c) Spinal segment consisting of two adjacent vertebrae and the interjacent IVD in a coordinate system indicating typical motions.

FIGURE 4 shows the normalized expression levels of microRNAs differently expressed in *P. acnes* positive and negative disc tissue: miR-574-3p, miR-29a-3p, miR-497-5p, miR-29c-3p, and miR-99b-5p.

FIGURE 5 shows Diagnostic miRNA score (DMS) values calculated for reference *P. acnes* positive and negative cases. Cut-off DMS value for *P. acnes* positivity is -0.01 (Left panel). ROC analysis of DMS values shows strong ability of DMS to distinguish cases with abundant *P. acnes* (culture and real-time PCR) and negative cases (Right panel).

FIGURE 6 shows DMS values calculated for independent validation with reference *P. acnes* positive and negative cases (Left panel). ROC analysis of DMS values shows strong ability of DMS to distinguish cases with abundant *P. acnes* (culture and real-time PCR) and negative cases (Right panel).

FIGURE 7 shows that disc tissue with positive culture for coagulase-negative staphylococci have DMS values characteristic for *P. acnes* negative samples (DMS > -0.01).

FIGURE 8 shows sequences of miRNAs deregulated in disc tissues with abundant *P. acnes.*

DETAILED DESCRIPTION OF THE INVENTION

The invention is defined by the appended claims.

In one aspect, the invention provides a method for detecting a low-virulence infection with *Propionibacterium acnes* in a patient, for example, by distinguishing true infections from non-infected samples, including from false positives and false negatives. The method comprises testing for the presence or amount of *Propionibacterium acnes* in a patient sample. Since testing for *Propionibacterium acnes* will lead to a large number of false-positives due to frequent sample contamination, the sample is also tested to discriminate true chronic infection from these false positives. In accordance with the invention, false positives are discriminated by evaluating an miRNA profile from the sample. As described herein, miRNA profiles of host tissue/cells are able to discriminate chronic low virulent infections, even though these low-grade infections cause only minor inflammatory responses on the local level.

As the term is used herein, a low-virulence infection is a chronic, low-grade, infection that is associated with a commensal microorganism. Exemplary commensal microorganisms include any of the commensal organisms that can be molecularly detected in host tissue samples, or determined by laboratory culture and include without limitation, *Propionibacterium sp.* (*P. acnes*) *Staphylococcus sp.* (e.g., coagulase negative staphylococcus, or *Staphylococcus aureus* or *Staphylococcus epidermidis), Corynebacterium, Lactobacillus sp., Pseudomonas sp.* (e.g., *Pseudomonas aeruginosa*), *Enterococcus sp., Streptococcus sp.* (e.g., *S. pneumoniae), Bacillus sp.* (e.g., *Bacillus cereus), Citrobacter sp., E. coli, Moraxella sp., Haemophilus sp., Neisseria sp., Clostridium sp., Enterobacter sp.,* and *Klebsiella sp.* The commensal microorganism may be poorly-culturable, or may be non-culturable. The microorganism may have a biofilm forming phenotype.

In some embodiments, the methods comprise detection or quantification of *Propionibacterium acnes* (*P. acnes*) in patient samples. *P. acnes* is a gram-positive aerotolerant anaerobe that forms part of the normal resident microbiota of the skin, oral cavity and the gastrointestinal and genito-urinary tracts. It is an opportunistic pathogen that has been linked to a wide range of infections and conditions, including implant infections, discitis, musculoskeletal conditions (e.g., osteitis, osteomyelitis, synovitis-acne-pustulosis-hyperostosis-osteitis (SAPHO) syndrome), sarcoidosis, chronic prostatitis, and prostate cancer.

Disclosed is determining the microbiome composition of a sample, including determining the relative or absolute abundance of commensal and/or pathogenic microorganisms in the sample. The microbiome composition of a sample can be determined, for example, by rRNA sequencing (e.g., 16S rRNA sequencing).

Low-virulence infection can be causative or a factor in any of a number of chronic conditions. The invention is applicable to any chronic condition in which a low-virulence infection is suspected. Thus, in some embodiments, the patient has a condition selected from chronic low back pain (CLBP), joint or cartilage inflammation, inflammation associated with an implant or prosthetic, endocarditis, potentially infected intravenous port system, or gastric ulcer. The patient may be a human or animal patient. Generally, the sample is a surgical sample or biopsy of the inflamed or damaged tissue, where a low-virulence infection is suspected. In some embodiments, the sample is from a site susceptible to colonization or infection by anaerobic commensal bacteria.

In some embodiments, the patient has chronic low back pain, and which is consistent with structural intervertebral disc damage and/or a low-virulence infection. In some embodiments, the patient may be a candidate for, and may be scheduled for, intervertebral disc surgery. Patients that have a low-virulence infection may be at increased risk of developing CLBP and/or may become "failed back surgery" patients, unless diagnosed correctly and treated appropriately. For example, some patients that undergo disc surgery will also suffer from CLBP prior to the acute condition necessitating their surgery. Also, a certain proportion (around 5 to 10%) of patients undergoing disc surgery will develop CLBP in the follow-up, which are sometimes referred to as "failed back surgery" patients or "post-discectomy syndrome". These conditions are statistically associated with a low-virulence infection.

Discitis could either very rapidly become clinically apparent and be diagnosed rather easily by the appropriate diagnostic measures or turn into a "lowgrade", "smoldering" infection, which can be extremely difficult to distinguish from certain degenerative processes that also can cause spinal pain. In the first case, a so-called "pyogenic infection" with clear local and systemic symptoms would result. Such a "high-grade" infection will frequently result in sepsis and even septic shock if left untreated. In the second case, the clinical features can be comparatively mild and unspecific such as local pain without a systemic reaction. Such phenomena are known also in the context of periprosthetic infections after arthroplasties of the shoulder, hip and knee joints. Specifically with such low-grade infections, the symptoms and complaints of the affected patients tend to be much more like those of degenerative or mechanical spinal problems than those typically associated with infection. This is especially so, because IVDs with preexisting degenerative changes such as nucleus dehydration and annular fissures would be more prone to colonization with a low-virulence infective agent as opposed to healthy IVDs.

Thus, in some embodiments, the patient has intervertebral disc disease. For example, the sample can be an intervertebral discectomy, which is obtained during surgery. In still other embodiments, the patient is scheduled for disc surgery, and a fine needle biopsy is isolated for testing prior to surgery. Exemplary disc surgeries include surgery to repair a disc herniation, primary lumbar fusion surgery, or primary disc arthroplasty. In these embodiments, the invention involves analysis of disc tissue for the presence of *P. acnes,* and/or for the presence of a miRNA signature indicative of a low-virulence infection with *Propionibacterium acnes.*

The invention involves detecting or quantifying *P acnes* in the disc tissue sample by microbiological cultivation or by genetic, immunochemical, or spectroscopic analysis. The invention further comprises evaluating the miRNA to classify the profile as being indicative of low virulence infection, or not being indicative of a low virulence infection.

In some embodiments, the presence of *Propionibacterium acnes* is evaluated by culture, including aerobic and/or anaerobic cultivation and subsequent biochemical and spectroscopic (e.g., MALDI-TOF MS) identification of species. For example, tissue samples are processed under sterile conditions, and can be processed by homogenizing (e.g., in sterile saline). Various homogenization techniques can be used, including sterilized sea sand to aid grinding in a laboratory mortar or Stomacher (Seward, UK). Homogenized samples are inoculated on agar surface for aerobic and anaerobic culture. For example, an aerobic culture can use Columbia Blood Agar (Oxoid) with 7% of sheep blood (CBA), and the anaerobic culture can employ Wilkins Chalgren Anaerobic Agar (Hi Media) with 7% of sheep blood and vitamin K (WCHA). Inoculated WCHA plates are incubated anaerobically (80% nitrogen, 10% $CO_2$ and 10% $H_2$) at 37°C for a minimum of 14 days. CBA plates are incubated aerobically at 37°C for about 7 days. In some embodiments, portions of the homogenized tissues are transferred into test tubes containing broth (e.g., 10 ml VL broth (Merck)) and subsequently overlaid with sterile paraffin oil to prevent access of oxygen and incubated at 37°C for about 7 days, and inoculated on WCHA in anaerobic conditions at 37°C for a minimum of 7 days. When the signs of microbial growth appear, the content of such tube is inoculated on CBA and WCHA and incubated as described above.

Obtained isolates can be sub-cultured on WCHA plates (incubation for 7 days, anaerobic atmosphere) and on CBA (incubation for 1 day, aerobic atmosphere) to obtain distinct colonies for further analysis. Isolates can be characterized by growth characteristics, by colony morphology, by Gram staining and by test catalase. Presumptive *P. acnes* isolates can be identified by biochemical analysis, for example, using the RapidID ANA II System (Remel) or by MALDI-TOF MS (microflex™ LT MALDI-TOF MS System + software + bacterial spectra library, Bruker Corp).

For molecular analysis, the sample may be a fresh tissue sample, or may be preserved, such as an FFPE sample or other preservation technique. In some embodiments, the sample is preserved or processed for molecular analysis by about 24 hours post-surgery or post-biopsy, or by about 48 hours, or by about 72 hours, or by about 96 hours post-surgery or post-biopsy.

For genetic analysis, nucleic acids (e.g., DNA and/or RNA) are isolated from the sample for analysis. In some embodiments where the sample comprises large amounts of cartilage (e.g., joint or disc tissue), the sample is processed by digestion with collagenase (e.g., Collagenase A) and/or proteinase (e.g., Proteinase K), or other enzymes useful for degrading the extracellular matrix.

In still other embodiments, immunochemistry (e.g., immunohistochemistry or ELISA) can be used to detect protein epitopes of *P. acnes.* Immunohistochemistry can be conducted on preserved or fresh tissue samples. For instance, without limitation, monoclonal antibodies against *P. acnes* can be used, such as antibodies specific for epitopes of cell-membrane-bound lipoteichoic acid (PAB antibody) and ribosome-bound trigger factor protein (TIG antibody).

Other techniques such as microscopy on tissue samples can be used to identify positive samples, and may employ any appropriate staining reagent (e.g., gram stain or fluorescent in situ hybridization (FISH)) or other immunoreagents specific for the commensal microorganism of interest. In some embodiments, microscopy is used to identify *P. acnes.* Application of FISH for visualization of *P. acnes* in tissue is described in Alexeyev OA, et al. Direct visualization of Propionibacterium acnes in prostate tissue by multicolor fluorescent in situ hybridization assay. J Clin Microbiol. 2007 Nov; 45(11):3721-8.

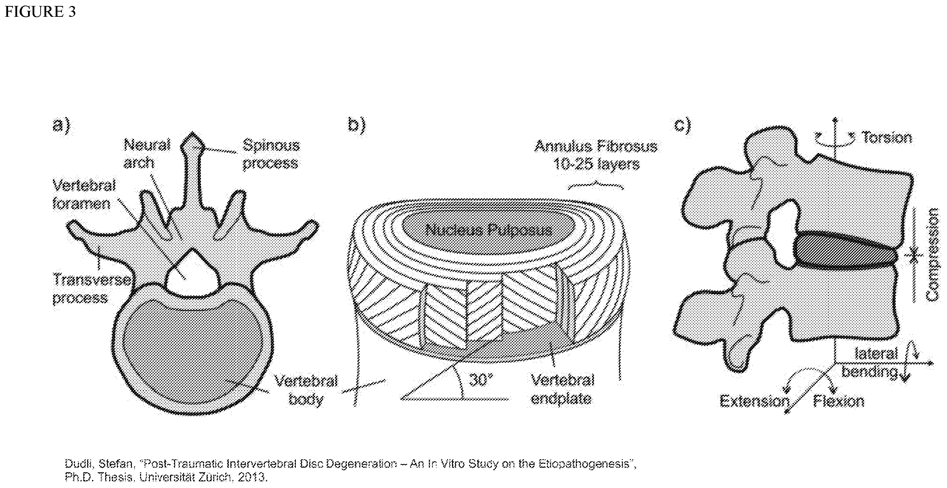

In some embodiments, nucleic acids are isolated from intervertebral disc (IVD) tissue, which may include nucleic acids of intracellular bacteria. The IVDs are the major mobile joints of the spine. They appear kidney shaped in the transversal plane and consist of three distinct structures: the central gelatinous nucleus pulposus (NP), the collagenous annulus fibrosus (AF), which surrounds the NP circumferentially, and the cartilage endplates (CEP), which separate the AF and NP from the vertebral bodies (Figure 3B and B). The IVD sided limits of the vertebral bodies are referred to as vertebral or bony endplates. They are composed of a layer of semi-porous thickened cancellous bone and form together with the CEPs the endplates (EPs). The IVD is comprised of an extensive extracellular matrix (ECM), which is maintained by cells with a tissue specific phenotype. They occupy less than 0.5% of the tissue volume.

The extracellular matrix comprises 99.5% of the IVD. The basic biochemical components of the NP, the AF and the CEP are the same, namely water, proteoglycans (PG) and collagens, but their relative proportions vary. The major PG of the IVD is aggrecan. It consists of a core protein to which up to 100 keratin and chondroitin sulfates are covalently bound. These are highly negatively charged glycosaminoglycans (GAG), which imbibe water and confer a viscoelastic behavior to the tissue, in particular to the highly hydrated NP. Collagen type I and II make up approximately 90% of total IVD collagen. In the NP, collagen fibers are irregularly oriented; in the AF, they are organized into 10-25 uni-directionally aligned lamellae, which encircle the NP and attach to the CEP in the inner zone and to the vertebral endplates in the outer zone (Figure 3B). The most abundant non-structural proteins in the IVD are the families of MMPs (matrixmetalloproteinases) and ADAMTS (A Disintegrin and Metalloproteinase with Thrombospondin Motifs). These are zinc-dependent proteinases, which can cleave almost all components of the ECM.

The cells of the IVD synthesize and maintain the extracellular matrix ("ECM"). They control the homeostasis between ECM synthesis and degradation. Compared to other tissues, relatively few cells have to maintain an extensive ECM. Consequently, the turnover of ECM proteins takes years. Three main types of cells are distinguished in the NP and AF: notochordal cells (NCs), NP and AF cells. NCs are remnants from the embryonic notochord and build the primary NP. In early childhood, NCs are replaced by NP cells. The AF and NP cells are of mesenchymal origin. According to their gene expression profile, AF cells are fibrocytic and NP cells chondrocytic. AF cells synthesize collagen I as the main structural protein, NP cells aggrecan and collagen II. All three cell types sustain a pericellular matrix, similar to the chondron of chondrocytes, with a composition distinct from the intercellular matrix.

In accordance with embodiments of the invention, *P.acnes* is cultured from cells of the NP, or nucleic acids are isolated for genetic analysis, or the cells of the NP are evaluated for bacterial antigens.

In some embodiments, the presence of the *P. acnes* is detected in cells from inflamed tissue. For example, intracellular presence of *P. acnes* supports its long-term persistency in the host, which could result in a chronic inflammatory state.

In some cases and for various reasons, attempts to establish a bacterial culture may be unsuccessful. Typical reasons for this are: (1) biofilm based orthopedic related infections are often culture negative [Achermann Y, et al. Propionibacterium acnes: from commensal to opportunistic biofilm-associated implant pathogen. Clin Microbiol Rev 2014;27:419-40]; (2) start of an antimicrobial therapy prior to the acquisition of the specimen; (3) biopsy did not yield enough tissue / colony-forming units (CFU); (4) biopsy was taken from the periphery / a non-representative area of the lesion; (5) culture media are inappropriate for the specific infectious agent (as is often the case with tuberculosis, other intracellular pathogens and certain anaerobic germs or fungi); (6) inappropriate transport conditions and delays until the specimen is processed in the lab; (7) reproduction rate under the laboratory conditions is very low (e.g.: tuberculosis); and (8) cultures are discarded too early, given the appearance of being sterile, in the microbiological lab and are not incubated for sufficient periods of time in search of the anaerobic germs. Many other potential reasons exist for the failure to establish a microbiological diagnosis, especially with low-grade disc infections, which often are caused by anaerobic germs.

In some embodiments, the presence or level of *P. acnes* is determined (alternatively or in addition to culturing) by hybridization or amplification of microbial nucleic acids. For example, detection assays include real-time or endpoint polymerase chain reaction (PCR), nucleic acid hybridization to microarrays, or nucleic acid sequencing. Detection assays can involve detection of genomic DNA, or RNA, including after reverse transcription in some embodiments. In some embodiments, the sample is subjected to "deep sequencing" to prepare an absolute or relative abundance of microbes present in the sample. Various sequencing strategies are known, including pyrosequencing (e.g., as available from 454 Life Sciences), nanopore sequencing, electronic sequencing (Genapsys, Inc., Redwood City, CA), and Sanger sequencing.

These molecular techniques are very sensitive and specific and do not suffer from many of the above-mentioned problems associated with classic microbiological culturing. However, because these techniques are very sensitive and because many commensal bacteria are ubiquitous, potential sample contamination is always a concern, and this can lead to an unacceptable rate of false positive results. Moreover, it was shown that *P. acnes* DNA is a frequent contaminant of commercial Taq polymerases and PCR solutions. See Lupan I, et al. The evidence of contaminant bacterial DNA in several commercial Tag polymerases. Rom Biotech Lett 2013;18:8007-12.

In some embodiments, molecular detection assays are conducted alongside culturing. Molecular assays, while capable of identifying a causative agent, do not necessarily provide data on the specific susceptibility or resistance of the causative agent to different antimicrobial drugs. Thus, culturing can provide additional information that is useful to initiate the optimal therapy.

In some embodiments, techniques such as microbial culture, PCR, microscopy and others, are used on a cohort of samples to identify *P. acnes*), and to identify a miRNA signature in the cohort that correlates to abundant levels of *P. acnes.*

The less virulent the ongoing infection, the more challenging the infection is to diagnose. These so-called low-grade infections cause significantly less inflammatory response on a local level and none or only very minor ones on a systemic level. This in turn negatively affects not only the capacity of imaging studies and laboratory tests to correctly diagnose such an infection, but more importantly, may already suppress the primary clinical suspicion of such an infection being present and the cause of a patient's symptoms. For example, the features of disc infections on MRI, the most commonly used advanced diagnostic imaging study beyond CR for low back pain, are often mild and non-specific. The most commonly observed changes would be a more or less pronounced bone marrow edema (equivalent to type 1-Modic changes) in the vertebral end plates adjacent to an infected disc, which is what can also be seen with certain common degenerative conditions such as activated osteochondrosis.

In some embodiments, the molecular detection assay detects microbial ribosomal RNA (rRNA) genes, such as 16S and/or 23S rRNA genes, and particularly the variable regions of 16S. For example, 16S rRNA sequencing can be used to characterize the complexity of microbial communities at body sites. 16S rRNA sequencing facilitates the recognition of microorganism taxa, e.g. species or higher taxonomic levels, and in cases of previously unknown microorganisms, allows for their general classification (e.g. families, phyla). Metagenomic whole genome shotgun (WGS) sequencing provides insights into the functions and pathways present in the microbial communities.

The 16S rRNA sequence contains both highly conserved and variable regions. These variable regions, nine in number (VI through V9), are used to classify organisms according to phylogeny, making 16S rRNA sequencing particularly useful in metagenomics to help identify taxonomic groups present in a sample. These sequences can be interrogated using chip technologies, RT-PCR, or deep sequencing.

In some embodiments, the presence of *P. acnes* is determined by hybridization-based assay, such as a microarray. For example, the PhyloChip approach in particular is a microarray-based method that identifies and measures the relative abundance of more than 50,000 individual microbial taxa. This approach relies on the analysis of the entire 16S ribosomal RNA gene sequence, which is present in every bacterial genome but varies in a way that provides a fingerprint for specific microbial types. The microarray-based hybridization approach ensures that measurements on important low abundance bacteria are not overwhelmed by commonplace, dominant microbial community members.

In some embodiments, the detection assay enables the typing *P. acnes* to help differentiate potential contamination, or to identify potential therapeutic agents that would likely be effective against the infection. In some embodiments, the method comprises typing of *P. acnes* in disc tissue by PCR or other molecular technique, and in derived *P. acnes* colonies. Where the *P. acnes* is not a result of contamination, the strain should be the same in both. Thus, cases that are positive with high levels of the same *P. acnes* strain by both cultivation and PCR will be considered a good reference and indicative as true positive for microRNA profiling.

Table 1: Exemplary Probes and Primers for *P. acne* detection

| RealTime PCR Assay | |
|---|---|
| 131-bp portion of *P. acnes* 16S rRNA | Forward: GCGTGAGTGACGGTAATGGGTA (SEQ ID NO:1) Reverse: TTCCGACGCGATCAACCA (SEQ ID NO:2) |
| EndPoint PCR Assay | |
| a 600bp region of 16S rRNA gene | Forward: GGGTTGTAAACCGCTTTCGCCT (SEQ ID NO:3) Reverse: GGCACACCCATCTCTGAGCAC (SEQ ID NO:4) |
| Multiplex PCR for typing *of P. acnes* strains | |
| 16S rRNA (All *P.acnes*) | Forward: AAGCGTGAGTGACGGTAATGGGTA (SEQ ID NO:5) |
| ATPase (Type IA1/IA2/IC) | Reverse: CCACCATAACGTGCTGGCAACAGT (SEQ ID NO:6) Forward: GCGTTGACCAAGTCCGCCGA (SEQ ID NO:7) |
| sodA (Type IA2/IB ) | Reverse: GCAAATTCGCACCGCGGAGC (SEQ ID NO:8) Forward: CGGAACCATCAACAAACTCGAA (SEQ ID NO:9) |
| Toxin, Fic family (Type IC) | Reverse: GAAGAACTCGTCAATCGCAGCA (SEQ ID NO:10) Forward: AGGGCGAGGTCCTCTTCTACCAGCG (SEQ ID NO:11) |
| atpD (Type II) | Reverse: ACCCTCCAACTGCAACTCTCCGCCT (SEQ ID NO:12) Forward: TCCATCTGGCCGAATACCAGG (SEQ ID NO:13) |
| recA (Type III) | Reverse: TCTTAACGCCGATCCCTCCAT (SEQ ID NO:14) Forward: GCGCCCTCAAGTTCTACTCA (SEQ ID NO:15) Reverse: CGGATTTGGTGATAATGCCA (SEQ ID NO:16) |
| Sequencing (recA to differentiate *P. acnes* strains) | |
| 1201 bp amplicon | AGCTCGGTGGGGTTCTCTCATC (-96 to -75) (SEQ ID NO:17) GCTTCCTCATACCACTGGTCATC (+1105 to +1083) (SEQ ID NO: 18) |
| FISH | |
| a Cy3-tagged nonsense EUB338 probe | 6-carboxyfluorescein (FAM)-tagged eubacterial EUB338 probe |
| Cy3.5-tagged probe specific for *P. acnes* 23 S rRNA | GAGTGTGTGAACCGA TCA TGT AGT AGGCAA (SEQ ID NO:19) |

In some embodiments, the molecular detection assay uses RealTime PCR to provide an absolute quantification of *P. acnes* gene copies, based on a calibration curve.

Where the detection of *P. acnes* is positive, whether conducted by one or more of culture, PCR, hybridization, microscopy, or immunochemistry, there is generally an unacceptably high rate of false positives, most likely due to frequent sample contamination by microflora. To discriminate these false positives, a miRNA profile for the sample is evaluated for the presence of a miRNA signature that is indicative of a low-grade or low-virulence infection. For example, RNA can be isolated from host cells in the sample, and the relative abundance of the miRNAs evaluated by any of several available miRNA detection platforms. These include Real-Time PCR (e.g., Exiqon; TaqMan Low-Density Arrays (TLDA) Human MicroRNA Panel; Life Technologies; QIAGEN SYBR® Green-based, real-time PCR profiling of miRNAs using the miScript PCR System), Microarray based platforms (e.g., Affymetrix GeneChip MiRNA; Agilent SurePrint Human miRNA Microarrays; Exiqon miRCURY LNA™ microRNA Arrays), Next-generation sequencing-based platforms (e.g., Illumina small RNA sequencing; IonTorrent small RNA sequencing), and in-situ microRNA hybridization in FFPE tissues. Generally, RNA profiles can be determined by amplification, hybridization, and/or sequencing technologies.

The miRNA signature may be correlative of a low-virulence infection, without regard to the bacterial species, or may be specific for a bacterial species or strain, such as *P. acnes,* coagulase negative staph, *E. coli,* or *Corynebacterium,* etc. A miRNA signature is determined by training a classifier algorithm with miRNA profiles from infected cells and non-infected cells. The cells may be infected in vitro, or may be in vivo infected cells (infected cells from clinical samples). In some embodiments, cells are infected with clinical isolates of *P. acnes* in vitro. In some embodiments, infected cultures are maintained for at least one day, at least one week, or at least two weeks, prior to determining the RNA profiles, to closely model chronic infection. Cells infected with a range of commensal pathogens may be used to prepare bacteria-specific and bacteria-non-specific signatures. In some embodiments, NP cells are cultured in vitro, and infected with commensal pathogens such as *P. acnes,* and the resulting RNA profile used to train a classifier algorithm that distinguishes *P. acnes-infected* cells from non-infected cells. Classifiers can be trained using *P. acnes* positive cells vs *P. acnes* negative cells; coagulase-neg Staphylococcus-positive cells vs. coagulase-negative Staphylococcus negative cells; and *P. acnes* and coagulase-neg. staph positive cells vs. cells negative for both.

In some embodiments, the RNA signature is trained using infected cells (e.g., *P acnes*-infected NP cells) and contaminated cells, to distinguish true chronic infection from acute "infection" due to contamination. Similarly, RNA signatures can be trained to differentiate organisms, such as bacteria, virus, fungi, and parasites, and in some embodiments, to differentiate gram positive vs. negative bacteria, and/or bacterial species or strains.

RNA signatures are trained using samples that test positive or abundant, versus negative or non-abundant, for the level of the commensal microorganism. In some embodiments, positive samples are abundant for the commensal microorganism (e.g., in at least the top 50%, 60%, 70%, 75%, 80%, or 90% of samples in a cohort) by at least one molecular technique (e.g., quantitative PCR) and optionally also by culture or microscopy. In some embodiments, positive samples produce at least about $10^3$ CFU per mL, or more than about $10^4$ CFU per mL by culture. Negative samples are generally negative by a molecular technique, such as quantitative PCR, or are in at least the bottom quartile, bottom 20% or bottom 10% quantitatively. Generally, negative samples are substantially negative by culture or microscopy. For example, in some embodiments, negative samples produce less than about $10^3$ CFU per mL by culture, or less than about $10^2$ CFU per mL, or less than about 10 CFU per mL. In this context, the culturing method may employ the process shown in Example 3. In some embodiments, the negative samples are in the bottom quartile, or bottom 20%, or bottom 10%, with regard to CFU/mL established by culture.

In some embodiments, RNA signatures from infected clinical samples will be trained from the results of metagenomic analysis, that is, using the absolute or relative abundance of microbes identified by deep sequencing or hybridization array.

Exemplary computational tools for distinguishing or classifying mRNA or miRNA signatures include Principal Components Analysis, Naive Bayes, Support Vector Machines, Nearest Neighbors, Decision Trees, Logistic, Artificial Neural Networks, and Rule-based schemes. The computer system may employ a classification algorithm or "class predictor" as described in R. Simon, Diagnostic and prognostic prediction using gene expression profiles in high-dimensional microarray data, British Journal of Cancer (2003) 89, 1599-1604. The classifier algorithm may be supervised, unsupervised, or semi-supervised.

In some embodiments, the classifier uses 1, 2, 3, 4, or 5 features (miRNAs), not including expression controls. In some embodiments, the algorithm uses from 2 to about 100 features (miRNAs) to comprise the signature. In some embodiments, the algorithm uses from 2 to about 50 features, or from 2 to about 30 features, or from 2 to about 20 features, or from 2 to about 10 features to comprise the signature. In some embodiments, the classifier is based on from 5 to 50 features, or from 10 to 50 features, or from 20 to 50 features. Exemplary human miRNAs are shown in Tables 2 and 8.

MicroRNAs (miRNAs) are important regulators of gene expression comprising an abundant class of endogenous, small noncoding RNAs (18-25 nucleotides in length). They are capable of either promoting mRNA degradation or attenuating protein translation. Bioinformatic studies have estimated that microRNAs may regulate more than 50% of all human genes and each miRNA can control hundreds of gene targets. Some miRNAs are expressed in a cell specific, tissue-specific and/or developmental stage-specific manner, while others are expressed ubiquitously. The number of verified miRNAs is still growing - the latest version of web-based database miRBase has annotated over 1800 precursor and 2342 mature sequences in the human genome. Based on the annotations for the genomic position of miRNAs which indicated that a vast majority of miRNAs are located in intergenic regions (>1 kb away from annotated or predicted genes), it has been postulated that most miRNA genes are transcribed as autonomous transcription units. MiRNAs may serve as master regulators of many fundamental biological processes, such as embryogenesis, organ development, cellular differentiation, proliferation, apoptosis, etc., affecting such major biological systems as stemness and immunity. Other small non-coding RNAs (e.g. piRNAs, snRNAs, snoRNAs, and circRNAs) or long non-coding RNAs (e.g. lncRNAs, lincRNAs, T-UCRs) may be used for detection of low virulent infections in accordance with the disclosure.

As a consequence, specific patterns of miRNA deregulation have been identified in variety of human cancers as well as pathologies of cardiovascular, urinary and other organ systems. In comparison to viral infections, miRNA response to bacterial pathogens has been less explored.

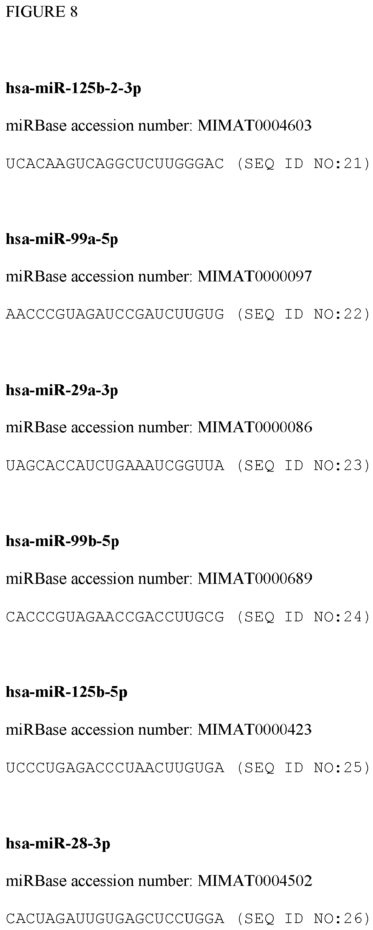

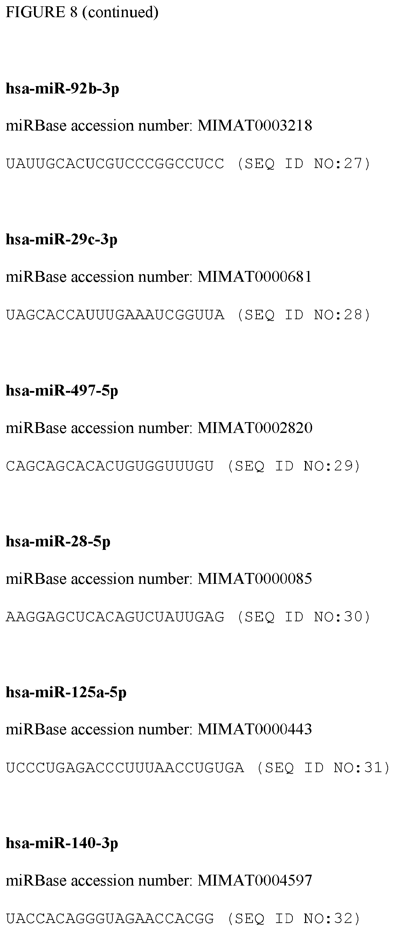

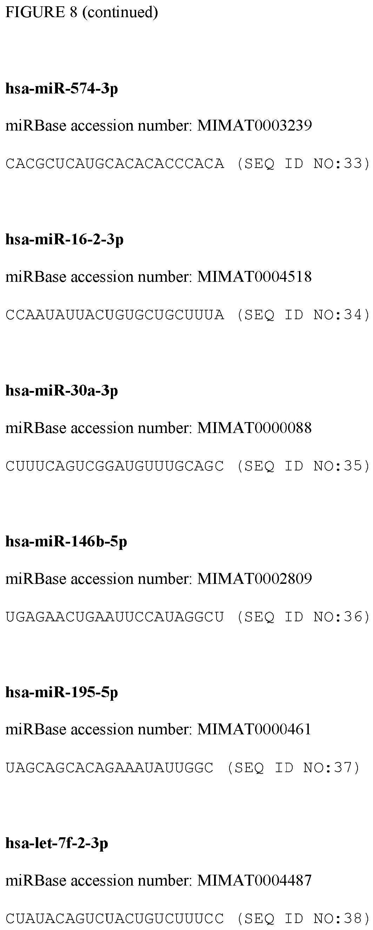

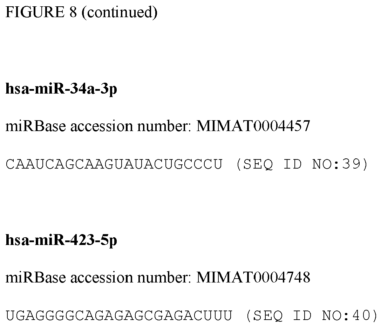

In some embodiments, the levels of from 2 to about 1000 RNAs (miRNAs) are detected in the RNA isolated from patient samples. For example, from about 2 to about 500, or from 2 to about 300, or from 2 to about 200, or from 2 to about 100, or 2 to 10 miRNAs are detected. In some embodiments at least 50 miRNAs are detected. In these or other embodiments, no more than 500, 300, or 100 or 10 or miRNAs are detected. In some embodiments, from 2 to about 5 or from 2 to about 10 miRNAs are individually detected, and the relative abundance determined with respect to controls. For example, miRNA signatures can be trained based on miRNA profiles for the miRNAs in Table 2, Table 4, Table 5, or the human miRNAs in Figure 2 or Figure 8.

In some embodiments, two or more of the following miRNAs are detected in patient samples (e.g., disc tissue): miR-574-3p, miR-29a-3p, miR-497-5p, miR-29c-3p, and miR-99b-5p. In some embodiments, miR-29a-3p and miR-574-3p are detected (e.g., in disc tissue). Expression may be quantified relative to the expression of one or more control genes, such as RNU38B and/or RNU48. Control RNAs can be selected to control for variation in the amount of starting material, sample collection, RNA preparation and quality, and reverse transcription (RT) efficiency. Normalization to endogenous control genes is an accurate method to correct for potential RNA input or RT efficiency biases. Other potential controls include housekeeping genes, such as ACTB (β-Actin) and GAPDH4. The endogenous control in some embodiments demonstrates gene expression that is relatively constant and highly abundant across tissues and cell types of interest. In some embodiments, a miRNA score is established based on the relative levels of expression for the miRNA features in positive and negative samples within the cohort, with the score distinguishing independent positive and negative samples. An exemplary diagnostic miRNA score (DMS) for distinguishing *P. acnes* positive disc tissue samples from *P. acnes* negative disc tissue samples is: DMS = 18.71 - 11.24 * log10 (miR-29a-3p) + 10.4 * log10 (miR-574-3p), where the cut-off is set within the range of 0.0 to -0.4 or 0.0 to -0.3 or 0.0 to -0.2 (less than or equal to the cut-off is *P. acnes* positive). For example, in some embodiments the cut-off is set at -0.01. In some embodiments, this score can substitute for microbial culture, and/or PCR (or other molecular assay). In some embodiments, the score is used to identify false positives identified through, for example, PCR or other molecular assay.

In cases where the diagnostic tests described herein confirm a low-virulence infection, the patient is recommended for treatment. In cases where a low-virulence infection is not confirmed (including false-positives detected by culture of molecular analysis), the patient is not recommended for treatment of a chronic infection.

In particular, where samples are positive for a low-virulence infection using a biopsy in advance of surgery, a local antibiotic or antiseptic rinse can be applied at the time of surgery. Alternatively, an oral, intravenous, or local antibiotic regimen can be administered prior to surgery.

When the sample is isolated during the surgery, and tests positive for a low-virulence infection, an oral, intravenous, or intervertebral antibiotic regimen can be administered during the days, weeks, or months post-surgery.

In some embodiments, the patient is determined to have a low virulence infection based on a biopsy sample. The patient may then be administered an antibiotic regimen, and where the low back pain is reduced or eliminated, surgery such as a discectomy or other invasive procedure may be avoided, for example, in favor of a less invasive procedure.

Where a tissue sample obtained during a routine disc surgery (discectomy) yields a positive test, antibiotic treatment may be provided, optionally guided by sensitivity testing when available and close clinical as well as imaging follow-up (MRI). Should the patient suffer from persistent or increasing low back pain and possibly even exhibit signs of progressive disc space inflammation after a microdiscectomy, the treating specialist could indicate for a complete discectomy and intervertebral arthrodesis in order to treat both, the infection and the back pain. Should the patient improve under antibiotic treatment, the antibiotic will be discontinued after a certain period of time with further clinical and imaging follow-up.

When the tissue sample is obtained by means of a percutaneous biopsy prior to a planned surgery yields a positive test, a targeted antibiotic pretreatment could be performed prior to the planned procedure. Depending on a potential clinical improvement under such treatment, a planned surgery could be changed to a less invasive procedure or to conservative treatment. During a surgery and with the knowledge of a lowgrade infection being present, topical antiseptics or antibiotics could be used in an attempt to increase the chances of resolving the infection and hence of a good clinical outcome.

Embodiments of the invention will now be described through the following examples.

EXAMPLES

Approximately 800,000 individuals undergo intervertebral disc surgeries in the U.S. each year to relieve intractable radicular (neuropathic) pain. The procedure involves removal of a portion of the disc causing inflammation of the nerve. The vast majority of these individuals have underlying degenerative disc disease, a condition that predisposes patients to chronic lower back pain, intervertebral disc herniation, sciatica, and other conditions causing significant disability and morbidity in our society. However, about 25% of these surgeries fail to return the patient to a healthy condition.

The cause of degenerative disc disease is often idiopathic, and there is growing evidence that a subset of patients undergoing intervertebral disc surgeries have a low virulence infection, potentially attributable to *Propionibacterium acnes* as well as *Corynebacterium* and *Staphylococcus.* It is believed that low virulence infection is a causative or compounding factor for some cases of degenerative disc, and/or a factor in the 25% of disc surgeries that fail to return a patient to a state of wellness. A low virulence infection is an infection that evades the immune system and can contribute to a chronic degenerative condition.

Example 1. microRNA (miRNA) in vitro Profiling

(1) NP cells derived from 3 samples of native disc tissue are infected by *P. acnes* (PA) (ATCC 6919, derived from facial acne), or PA derived directly from infected disc tissues, and with other bacterial species (e.g., *E. coli, Staphylococcus, Corynebacterium).*

(2) Bacterial intracellular occurrence is evaluated by cytokine response and DAPI staining at various time-points.

(3) Identify microRNA profile induced by bacteria in intracellular space. A first profile (Profile 1a) is the profile common for all bacterial species, and a second profile (Profile 1b) is the profile specific for *P. acnes,* or *E. coli* etc., for detection of contamination in tissue samples.

(4) Identify microRNA profiles (Profile 1a and Profile 1b) in different time points after infection of NP cell cultures. 30-60 minutes may be used as a model for acute infection, to identify profiles indicative of contamination which occur prior to sample fixation (e.g., in-vitro contamination signal). Long-term incubation (e.g., 1, 7 and 21 days) will be used to model chronic infection, and identify profiles indicative of chronic infection.

Global microRNA expression profiling (754 microRNAs) will be performed with Exiqon microRNA Human panel V3.0.

Example 2. miRNA Fresh Intervertebral Disc Tissue Profiling

(1) Enrollment of ~500 patients undergoing disc surgery in Czech Republic, with clinical data collection at the time of surgery and follow-up in 6 weeks, and 6 and 12 months.

(2) Divide each tissue sample into 3 portions:

1. For DNA purification and qPCR evaluation of bacterial DNA event. For Metagenomic approach, DNA is stored at -80°C immediately after extraction.

2. For RNA purification and qPCR evaluation of microRNA profiles. RNA is collected with stabilizing solution (RNAlater).

3. For microbiological evaluation, tissue will be transported at room temperature to Microbiology department as soon as possible.
For future research, blood plasma and urine are also collected and stored at - 80° C.

(3) Sample 1 will be used for DNA purification and in all samples will be quantified for *P. acnes* DNA by qPCR. In addition, other bacterial species, such as staphylococci, can be quantified.

(4) In samples positive by both cultivation and qPCR, PA strain will be identified in colonies and disc tissue by multiplex PCR and/or sequencing. To rule out contamination the same strain should be identified. Samples which are positive by cultivation and qPCR for the same strain will be considered as reference positive for microRNA profiling.

(5) Samples negative by cultivation and negative (or low level positive in case of no negative samples) by qPCR (e.g., PA, staphylococci) will be considered as reference negative for microRNA profiling.

(6) 20 PA-positive, 10 staphylococci-positive, and 30 negative will be used for microRNA profiling.

(7) By comparison of microRNA profiles from 20 PA together with 10 staph, versus 30 negative samples, a common microRNA profile associated with occurrence of bacteria in disc tissue is identified. By comparison of 20 PA+ and 10 staph+ versus negative samples separately, bacteria-specific profiles are determined.

(8) Comparison will be performed between in vitro microRNA signatures and in vivo microRNA signatures.

Example 3. Cross-Validation of Positivity in the Whole Cohort

In 500 samples there will be: N patients positive by cultivation, and N1 positive by qPCR. A diagnostic algorithm based on identified microRNA signatures will be developed. N3 patients will be positive by miRNA signature.

In cases positive for common miRNA signature, PA-specific and staph-specific miRNA signatures will be evaluated. From N3 positive for common signature: N4 will be positive for PA-specific miRNA signature; N5 will be positive for Staph-specific miRNA signature; N6 will be negative for both. N6 will be subjected to metagenomic analysis.

The following observations are expected: a higher frequency of true positivity in patients undergoing surgery with history of CLBP, and higher frequency of true positivity in patients with failed back surgery and especially in those who will suffer with CLBP (after evaluation of clinical outcome at 6 and 12 months).

Example 4. Establishment of Diagnostic microRNA Score (DMS)

*Patient cohort and definition of reference P. acnes samples*

326 patients (186 males and 140 females) were prospectively enrolled with an average age of $44 \pm 13$ years. None of the patients developed clinically evident post-operative discitis. As gold standard, quantitative bacterial culture was performed to determine *P. acnes* counts, and real-time PCR was performed to detect genome counts in disc tissue specimens. Procedures used for bacterial culture and real-time PCR are described in detail in Methods. One hundred thirty cases (40%) were *P. acnes* positive by culture. *P. acnes* counts ranged from 100 to 9000 CFU/ml with a median of 400 CFU/ml. By real-time PCR, *P. acnes* genomes were undetectable in 98 cases (30%). The number of *P. acnes* genomes in the 228 *P.* acnes-positive disc tissues ranged from 2 to 4531 with a median of 256 genomes per 500 ng of total DNA. Disc tissue samples characterized by abundant *P. acnes* by both, culture ($\geq 10^3$ CFU/ml; 75th percentile) and by real-time PCR ($\geq 500$ genomes in 500 ng of DNA; 75th percentile), were considered as reference *P. acnes* positive cases (N=45, 14%). On other hand, samples negative by both bacterial culture and real-time PCR were used as reference *P. acnes* negative cases (N=72, 22%). These *P. acnes* reference cases were used for global microRNA expression profiling, identification of diagnostic microRNAs, as well as validation and development of Diagnostic MicroRNA Score (DMS) in the 3-phase biomarker study.

Reference samples (45 positive, 72 negative) were further divided into discovery, training and validation sets proportionally to their *P. acnes* status. As there is a high-risk of contamination-based false positivity in non-abundant *P. acnes* positive cases, in the validation cohort also the samples (N=44) with non-abundant *P. acnes* (<$10^3$ CFU/ml) and/or various genome counts by real-time PCR (inconclusive results by standard method) were included to be evaluated by implementation of newly developed DMS. Patient's characteristics in studied cohorts are summarized in Table 3. In addition to these 161 patients, another 10 cases with positive disc culture for coagulase-negative staphylococci (CoNS) were included as a specificity control group in the validation part of the study.

Table 3. Patient characteristics (N=161)

| Parameters | Discovery set N=24 | Training set N=35 | Validation set N=102 |
|---|---|---|---|
| **Gender** | | | |
| Male | 16 (66 %) | 21 (60 %) | 64 (63 %) |
| Female | 8 (34 %) | 14 (40 %) | 38 (37 %) |
| **Age** | | | |
| Mean $\pm$ SD | $43 \pm 14$ y | $45 \pm 13$ y | $46 \pm 12$ y |
| **Previous spinal surgery** | | | |
| Yes | 2 (8 %) | 5 (13 %) | 9 (9 %) |
| No | 22 (92 %) | 30 (87 %) | 93 (91 %) |

(continued)

| Prior epidural injection | | | |
|---|---|---|---|
| Yes | 0 (0 %) | 4 (11 %) | 2 (2 %) |
| No | 100 (100%) | 31 (89 %) | 100 (98 %) |
| **Type of herniation** | | | |
| Protrusion | 2 (8 %) | 3 (9 %) | 3 (3 %) |
| Extrusion | 10 (42 %) | 14 (40 %) | 38 (37 %) |
| Sequestration | 12 (50 %) | 18 (51 %) | 61 (60 %) |
| **Intervertebral level** | | | |
| L2/L3 | 0 (0 %) | 0 (0 %) | 1 (1 %) |
| L3/L4 | 1 (4 %) | 1 (3 %) | 2 (2 %) |
| L4/L5 | 8 (34 %) | 16 (46 %) | 40 (39 %) |
| L5/S1 | 15 (62 %) | 18 (51 %) | 59 (58 %) |
| **P. acnes culture results*** | | | |
| > $10^3$ CFU/ml | 12 (50 %) | 15 (43 %) | 18 (18 %) |
| < $10^3$ CFU/ml | 0 | 0 | 39 (38 %) |
| negative | 12 (50 %) | 20 (57 %) | 45 (44 %) |
| **P. acnes genome counts** | | | |
| > 500 in 500ng DNA | 12 (50 %) | 15 (43 %) | 27 (26 %) |
| < 500 in 500ng DNA | 0 | 0 | 13 (13 %) |
| negative | 12 (50 %) | 15 (57 %) | 62 (61 %) |

*There was no co-infection observed in any case by culture.

*Discovery phase - miRNA deregulated in P. acnes positive and negative samples*

Expression profiling of 754 microRNAs in 12 reference *P. acnes* positive disc tissues and 12 *P. acnes* negative samples was carried out by use of Exiqon real-time PCR based technology. Only miRNA with average Ct (threshold cycle) lower than 35 in one group were statistically evaluated. MicroRNA expression levels were normalized to RNU38B. 20 microRNAs were differentially expressed in *P. acnes* positive and negative disc tissue samples (summarized in Table 4) as described in the Methods.

Table 4. MicroRNAs identified to be differentially expressed in disc tissues with abundant *P. acnes* by culture and real-time PCR (p-value<0.1 and adj. p-value<0.15).

| miRNA | Fold change | P-value | Adjusted p-value* |
|---|---|---|---|
| hsa-miR-125b-2-3p | 1.66 | 0.002 | 0.056 |
| hsa-miR-99a-5p | 1.5 | 0.003 | 0.056 |
| hsa-miR-29a-3p | 1.45 | 0.009 | 0.076 |
| hsa-miR-99b-5p | 1.49 | 0.009 | 0.076 |
| hsa-miR-125b-5p | 0.78 | 0.017 | 0.105 |
| hsa-miR-28-3p | 1.41 | 0.020 | 0.105 |
| hsa-miR-92b-3p** | 1.46 | 0.023 | 0.107 |
| hsa-miR-29c-3p | 1.58 | 0.039 | 0.112 |
| hsa-miR-497-5p | 1.82 | 0.039 | 0.112 |
| hsa-miR-28-5p | 1.35 | 0.039 | 0.112 |
| hsa-miR-125a-5p | 0.48 | 0.043 | 0.120 |
| hsa-miR-140-3p | 1.52 | 0.045 | 0.121 |
| hsa-miR-574-3p | 0.54 | 0.053 | 0.130 |
| hsa-miR-16-2-3p** | 1.38 | 0.061 | 0.131 |
| hsa-miR-30a-3p | 1.44 | 0.061 | 0.131 |
| hsa-miR-146b-5p | 0.62 | 0.071 | 0.139 |

(continued)

| miRNA | Fold change | P-value | Adjusted p-value* |
|---|---|---|---|
| hsa-miR-195-5p | 1.86 | 0.082 | 0.140 |
| hsa-let-7f-2-3p** | 1.44 | 0.098 | 0.141 |
| hsa-miR-34a-3p** | 1.8 | 0.099 | 0.145 |
| hsa-miR-423-5p | 2.05 | 0.100 | 0.149 |

\* Benjamimi-Hochberg correction for multiple hypothesis testing

\*\* MicroRNAs not selected for validation due to extremely low expression levels (Ct >33 in both groups)

*Training phase - validation and establishment of Diagnostic MicroRNA Score (DMS)*

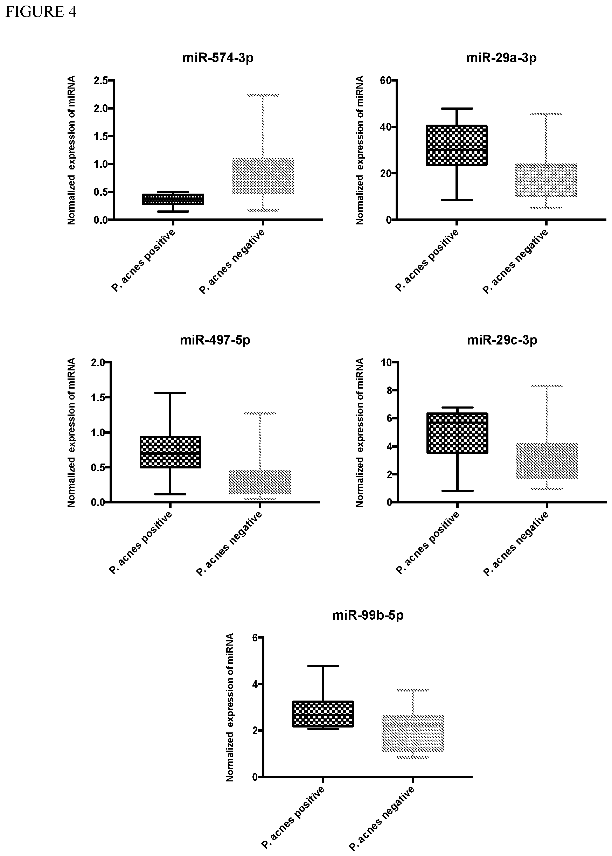

By use of individual microRNA expression assays (Life Technologies) and assays for 2 reference genes we determined expression levels of 16 candidate miRNAs from discovery phase and RNU38B and RNU48 in 35 disc tissue samples (15 reference *P. acnes* positive cases, and 20 reference negative cases) as described in the Methods. MicroRNA expression levels were quantified relatively to the average of two reference genes (RNU38B and RNU48). All samples passed quality control with Ct(RNU38B)<34 and/or Ct(RNU48)<31. Five microRNAs were confirmed to have significantly different expression levels in *P. acnes* positive and negative disc tissue samples, two of them remain significant even after adjustment of P-value for multiple hypothesis testing (Table 5, Figure 4).

Table 5. Independent validation of candidate microRNAs identified in discovery phase.

| miRNA | Fold change | P-value | Adjusted p-value* |
|---|---|---|---|
| **miR-574-3p** | **0.51** | **0.002** | **0.024** |
| **miR-29a-3p** | **1.87** | **0.002** | **0.031** |
| miR-497-5p | 2.79 | 0.005 | 0.063 |
| miR-29c-3p | 1.57 | 0.029 | 0.322 |
| miR-99b-5p | 1.37 | 0.047 | 0.437 |
| miR-195-5p | 1.85 | 0.100 | 0.687 |
| miR-28-5p | 1.48 | 0.105 | 0.687 |
| miR-30a-3p | 1.36 | 0.112 | 0.687 |
| miR-146b-5p | 1.50 | 0.184 | 0.803 |
| miR-34a-3p | 1.36 | 0.303 | 0.920 |
| miR-140-3p | 1.29 | 0.331 | 0.920 |
| miR-125a-5p | 0.74 | 0.387 | 0.920 |
| miR-423-5p | 1.46 | 0.389 | 0.920 |
| miR-125b-5p | 1.25 | 0.397 | 0.920 |
| miR-28-3p | 1.30 | 0.461 | 0.920 |
| miR-125b-2-3p | 0.90 | 0.625 | 0.920 |

\*Benjamimi-Hochberg correction for multiple hypothesis testing

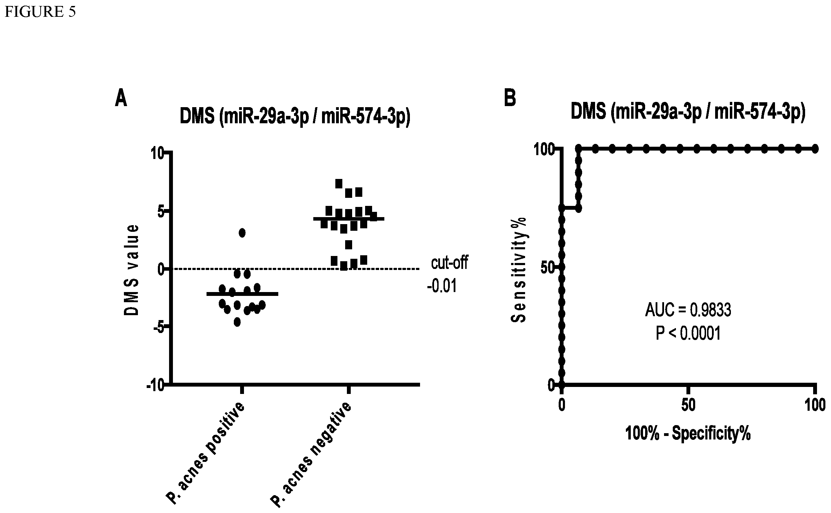

Two microRNAs (miR-29a-3p and miR-547-3p) were used for establishment of diagnostic microRNA score (DMS) (Formula 1). When applied to ROC analysis the cut-off value -0.01 was identified as the best discriminator between reference *P. acnes* positive and negative cases (Figure 5, AUC=0.9833).

miR-29a-3p and miR-547-3p expression levels are expressed relatively to the average of two reference genes (RNU38B and RNU48):

$$miR\text{-}29a\text{-}3p = 2^{-(Ct(miR\text{-}29a\text{-}3p)-average(Ct(RNU48)\ and\ Ct(RNU38B)))}$$

$$miR\text{-}574\text{-}3p = 2^{-(Ct(miR\text{-}574\text{-}3p)-average(Ct(PNU48)\ and\ Ct(RNU38B)))}$$

Formula 1:

$$DMS = 18.71 - 11.24 * \log10 \, (miR\text{-}29a\text{-}3p) + 10.4 * \log10 \, (miR\text{-}574\text{-}3p)$$

As shown in Figure 5, ROC analysis of DMS values shows strong ability to distinguish cases with abundant *P. acnes* (culture and real-time PCR) and negative cases.

*Validation phase - independent validation of DMS and its application in positive cases with non-abundant P. acnes* (inconclusive by standard methods)

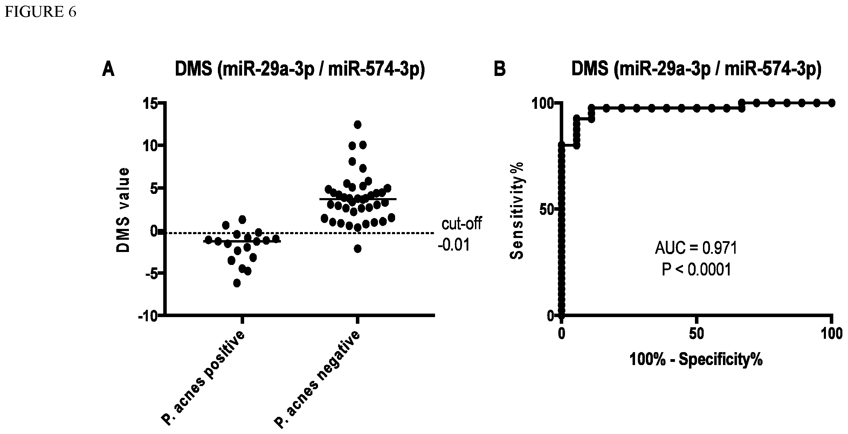

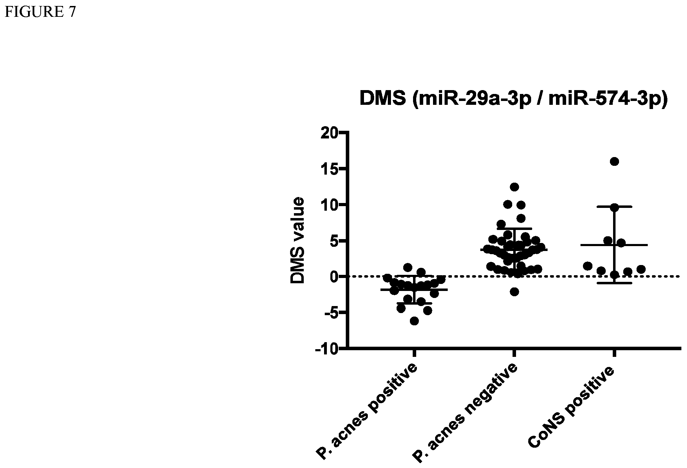

By use of individual microRNA expression assays (Life Technologies) and assays for reference genes, expression levels of miR-29a-3p and miR-574-3p were determined, constituting DMS and RNU38B and RNU48 similarly to the training phase. Application of DMS in 58 independent cases (18 reference *P. acnes* positive cases, 40 reference negative cases) confirmed its high analytical performance showing 95% accuracy in classification of the samples accordingly to their *P. acnes* status (Tables 6 and 7, Figure 6). Moreover, 10 disc tissue samples with coagulase-negative staphylococci (CoNS) positive culture were DMS-negative indicating specificity of DMS for *P. acnes,* or at least showing that DMS is not affected by the presence of the second most frequently observed microorganism (CoNS) in the disc tissue (Figure 7).

Addition of DMS to standard diagnostic methods (culture and real-time PCR) shows that almost 60% of the disc samples evaluated as positive with non-abundant *P. acnes* by culture and various genome counts by real-time PCR are false positives (Table 6).

Table 7. Diagnostic performance of 2-miRNA based DMS.

| | Training set | Validation set |
|---|---|---|
| AUC | 0.98 | 0.97 |
| Sensitivity | 0.93 | 0.89 |
| Specificity | 1.00 | 0.98 |
| Accuracy | 0.97 | 0.95 |
| PPV[#] | 1.00 | 0.94 |
| NPV[##] | 0.95 | 0.95 |

[#]PPV- positive predictive value, [##]NPV - negative predictive value DMS calculations are exemplified as follows.

Sample ID 4 - POSITIVE

Ct (miR-29a-3p) =25.02; Ct (miR-574-3p) =31.35; Ct(RNU38B) =31.58; Ct (RNU48) =28.76

Quality control: Ct (RNU38B) < 34 and Ct (RNU48) < 31 => RNA sample is VALID!

miR-29a-3p = $2^{-(25.02-(28.76+31.58)/2)}$ = 35.5062

miR-574-3p = $2^{-(31.35-(28.76+31.58)/2)}$ = 0.4414

DMS (id 4) = 18.71 - 11.24 * log10(35.5062) + 10.4 * log10(0.4414) = - 2.41

DMS (id 4) = -2.41 < DMS (positivity cut-off) = -0.01 = > sample is P. acnes POSITIVE

Sample ID 30 - NEGATIVE

Ct (miR-29a-3p) =26.49; Ct (miR-574-3p) =31.73; Ct(RNU38B) =32.43; Ct (RNU48) =30.07

Quality control: Ct (RNU38B) < 34 and Ct (RNU48) < 31 => RNA sample is VALID!

$$\text{miR-29a-3p} = 2^{-(26.49-(30.07+32.43)/2)} = 27.0959$$

$$\text{miR-574-3p} = 2^{-(31.73-(30.07+32.43)/2)} = 0.7170$$

$$\text{DMS (id 30)} = 18.71 - 11.24 * \log10(27.0959) + 10.4 * \log10(0.7170) = 1.1$$

DMS (id 30) = 1.1 > DMS (positivity cut-off) = -0.01 = > sample is P. acnes NEGATIVE

As shown in Table 6, 23 (59 %) out of 39 cases POSITIVE by culture with non-abundant *P. acnes* were evaluated as *P. acnes* NEGATIVE by implementation of DMS.

Four of 5 cases (80%) negative by culture but strongly POSITIVE by real-time PCR were assessed as NEGATIVE by DMS, one case (20%) was positive by DMS.

*Potential clinical implications*

If *P. acnes* infection of the disc is used for clinical decision making and standard methods applied for its diagnosis (bacterial culture and/or real-time PCR), more than one third of patients will receive potentially harmful antibiotic treatment without being infected. DMS with its analytical performance could fully substitute standard diagnostic techniques, which are time-consuming and highly vulnerable for contamination, and so provide solution how to prevent these unnecessary harmful treatments in patients with spinal diseases.

*Methods*

*Patients:* Inclusion criteria included: lumbar or lumbosacral radiculopathy with or without sensory deficits but with either a matching, clinically relevant motor deficit in correlating lumbar or sacral nerve root distributions (see imaging criteria) or with radicular pain (sciatica or femoralgia) that was intractable by conservative means; matching physical examination findings including positive straight leg raise test, dermatomal sensory deficits, myotomal motor deficits and/or a diminished deep tendon reflexes; current magnetic resonance imaging or computed tomography imaging of the lumbosacral spine showing a free nucleus pulposus sequestration or a disc herniation / protrusion in a distribution correlating with the clinically affected nerve roots and with the physical examination. Exclusion criteria included: coexistent infection or immunologically compromised conditions; corticosteroid or antibiotics use in the month before surgery; trauma; unknown radiographic mass; diagnosis of inflammatory arthritis or other rheumatologic diseases. The following epidemiological and clinical data were collected: Gender, age, intervertebral segment involved, type of herniation, prevalence of previous spinal surgeries, prior epidural steroid injections, and development of post-operative discitis. A written informed consent was obtained from each patient. The study was approved by the Institutional Review Board.

*Collection of intraoperative samples.* The surgical site was scrubbed with triple preparation of povidone iodine and draped using standard sterile technique. Standard perioperative antibiotics were given before the skin incision in all cases. Cefazolin was the standard antibiotic given in most cases. In penicillin-allergic patients, either vancomycin or clindamycin was administered. The precise location for the skin incision was guided by intraoperative fluoroscopy and a posterior midline approach using sharp dissection and electrocautery was performed. After placement of a self-retaining retractor (Caspar type) and under an operating microscope, ligamentum flavum was resected as required by means of Penfield dissectors and Kerrison rongeurs. The disc herniation was exposed by gentle retraction of the traversing nerve root and then removed together with the remnant loose fragments of the nucleus pulposus in the disc space near the annular defect. All tissue samples were handled in such a way as to minimize their contamination, retained in a closed sterile sample cup, and then passed off to the field for labeling and transport to the laboratory, where the disc tissue samples were further analyzed. The sample sizes were approximately 3x3x5-10x5x5 mm and not measured accurately as we attempted to obtain as much material as possible from the surgical specimen. Samples were not frozen prior to processing and culture was established within 2 to 4 hours post-surgery.

*Microbiological culture.* Fresh disc tissue samples were cut into smaller fragments using a sterile, individually packaged, gamma-irradiated scalpel; and, a sterile, gamma-irradiated petri dish. One of these fragments was placed into a 2 mL microcentrifuge DNA-free tube and stored at -80°C until processed for *P. acnes* DNA analysis. Tissue processing and homogenization was carried out in a sterile pestle and mortar with sterile quartz sand (size particle 0.1-0.5 mm; Penta, Czech Republic) and saline solution in aseptic conditions, in a class 2 biological safety cabinet. The homogenized tissue samples were inoculated onto Wilkins Chalgren Anaerobic Agar (Hi Media Laboratories, India) with 7% of sheep's blood and vitamin K. Inoculated plates were incubated anaerobically (80% nitrogen, 10% $CO_2$ and 10% $H_2$) in an Anaerobic Work Station (Ruskinn Technology, UK) at 37°C for 14 days and assessed for bacterial growth. The quantity of bacteria in the sample was expressed as colony forming units (CFU) in 1 ml of the homogenate. Identification of bacteria was carried out biochemically using the Rapid ANA II System (Remel, USA) and by MALDI-TOF (microflex™ LT MALDI-TOF System + software + bacterial spectra library, Bruker Corp.).

*DNA isolation.* Frozen tissue samples were thawed (median wet weight was 130 mg, range 20 - 180 mg), cut into small fragments and transferred to a sterile 2 mL microcentrifuge tube by use of newly opened sterile sets of needle, scalpel and tweezers. Small fragments of the tissue samples were further suspended in 500 µl of ATL buffer (Qiagen, Germany) with 50 µl of proteinase K (20 mg/ml) (Qiagen) and digested at 56°C and 650 rpm in a thermomixer overnight. To each set of the samples that were processed in parallel, a tube with sterile water was used as a laboratory contamination control to follow the entire laboratory process from digestion of the tissue and DNA isolation to real-time PCR analysis. DNA was extracted by use of the QIAamp UCP Pathogen Mini Kit (Qiagen) as described in the manufacturer's instructions. Concentration of DNA were measured spectrophotometrically using a Nanodrop 2000 (Thermofischer, USA); or with fluorescent dye and a Qubit 3.0 fluorometer (Life Technologies, USA) for samples with DNA concentrations less than 5ng/µl.

*P. acnes quantification by real-time PCR.* A previously described real-time PCR assay was performed using primers to amplify a 131-bp region of the 16S rRNA gene of *P. acnes:* forward primer 5'- GCGTGAGTGACGGTAATGGGTA - 3' (SEQ ID NO:1), reverse primer 5'-TTCCGACGCGATCAACCA-3' (SEQ ID NO:2) and TaqMan probe 5'-AGCGTTGTCCGGATTTATTGGGCG-3' (SEQ ID NO:20). The 15-µl PCR reaction mixture contained 6,75 µl of DNA sample, 5 pmol of each primer and 2 pmol of TaqMan probe, and IX TaqMan Gene Expression Master Mix (Life Technologies, USA). The QuantStudio 12K Flex system (Life Technologies, USA) was used with the thermal cycling profile of 50°C for 2 min, 95°C for 10 min and 50 cycles of 95°C for 15 s and 60°C for 1 min. The *P. acnes* genome equivalents in samples were estimated with an internal standard curve prepared with five replicates of six concentrations ($10$-$10^6$ copies) of synthesized *P. acnes* amplicon (131 bp) (Integrated DNA Technologies, USA). Laboratory contamination controls described above and PCR negative controls were included in every PCR reaction. Assays were done in duplicate for each sample, and the mean number of the 16S rRNA gene copies was calculated. To eliminate laboratory contamination, 16S rRNA counts detected in laboratory contamination control were subtracted from the copies number in the tissue samples. The number of bacterial genomes in each sample was finally calculated using the known number of copies of the 16S rRNA operon (3 copies/cell) in *P. acnes* and represented as the number of bacterial genomes in 500 ng of total DNA extracted from the disc tissue sample. Human β-globin gene was included as an internal control to allow assessment of the specimen quality and the nucleic acid extraction as well as the inhibition amplification process.

*RNA isolation.* Frozen tissue samples were thawed (median wet weight was 100 mg, range 20 - 145 mg), cut into small fragments and transferred to a sterile 2 mL microcentrifuge tube by use of sterile sets of needle, scalpel and tweezers. Small fragments of the tissue samples were further suspended in 500 µl of ATL buffer (Qiagen, Germany) with 50 µl of proteinase K (20 mg/ml) (Qiagen) and digested at 56°C and 650 rpm in a thermomixer overnight. Total RNA, including microRNAs and other small RNAs, was extracted by use of miRNeasy Mini Kit (Qiagen) as described in the manufacturer's instructions. Concentration of RNA were measured spectrophotometrically using a Nanodrop 2000 (Thermofischer, USA); or with fluorescent dye and a Qubit 3.0 fluorometer (Life Technologies, USA) for samples with RNA concentrations less than 5ng/µl.

*MicroRNA expression profiling.* Expression profiling of miRNAs was performed using microRNA Ready-to-Use PCR Panels (Exiqon, Vedbaek, Denmark). A set of two cards (Human Panel I+II, V4.M) enabling quantification of 752 human miRNAs and 6 endogenous controls for data normalization was used. First-strand cDNA synthesis was performed according to the standard protocol using Universal cDNA Synthesis Kit (Exiqon, Vedbaek, Denmark) and 100 ng of total RNA. Subsequently, real-time PCR amplification was carried out using ExiLENT SYBR Green master mix and PCR amplification primers that are microRNA specific and optimized with LNA™ (Exiqon, Vedbaek, Denmark). The final measurement was performed using a QuantStudio 12K Flex system (Life Technologies, USA).

*Individual microRNA expression analysis.* Complementary DNA was synthesized from total RNA using gene-specific primers according to the TaqMan MicroRNA Assay protocol (Applied Biosystems, Foster City, CA, USA). For reverse transcriptase reactions 10 ng of RNA sample, 50 nM of stem-loop RT primer, $1 \times$ RT buffer, 0.25 mM each of dNTPs, 3.33 U·µl$^{-1}$ MultiScribe reverse transcriptase and 0.25 U·µl$^{-1}$ RNase inhibitor (all from TaqMan MicroRNA Reverse Transcription kit, Applied Biosystems, Foster City, CA, USA) were used. Reaction mixtures (10 µl) were incubated for 30 min at 16 °C, 30 min at 42 °C, 5 min at 85 °C and then held at 4 °C (T100™ Thermal Cycler; Bio-Rad, Hercules, CA, USA). Real-time PCR was performed using the QuantStudio 12K Flex Real-Time PCR system (Applied Biosystems, Foster City, CA, USA). The 20-µl PCR reaction mixture included 1.33 µl of RT product, $1 \times$ TaqMan (NoUmpErase UNG) Universal PCR Master Mix and 1 µl of primer and probe mix of the TaqMan MicroRNA Assay kit (Applied Biosystems, Foster City, CA, USA). Reactions were incubated in a 96-well optical plate at 95 °C for 10 min, followed by 40 cycles at 95 °C for 15 s and 60 °C for 1 min. Following microRNA assays were used: miR-574-3p (ID: 002349), miR-29a-3p (ID: 002112), miR-497-5p (ID: 001043), miR-29c-3p (ID: 000587), miR-99b-5p (ID: 000436), miR-195-5p (ID: 000494), miR-28-5p (ID: 000411), miR-30a-3p (ID: 000416), miR-146b-5p (ID: 001097), miR-34a-3p (ID: 002316), miR-140-3p (ID: 002234), miR-125a-5p (ID: 002198), miR-423-5p (ID: 002340), miR-125b-5p (ID: 000449), miR-28-3p (ID: 002446), miR-125b-2-3p (ID: 002158). Two assays were used for quantification of reference genes: RNU38B (ID: 001004) and RNU48 (ID: 001006).

*Data analysis.* The threshold cycle data were calculated by SDS 2.0.1 software (Applied Biosystems, Foster City, CA, USA). All individual microRNA real-time PCR reactions were run in triplicates. The average expression levels of all measured miRNAs were normalized using RNU38B in cases global microRNA expression profiling and average of RNU38B and RNU48 in individual microRNA measurements. Expression levels were subsequently analyzed by the $2^{-\Delta Ct}$ method. RNU38B (SNORD38B) was selected as an endogenous control through combination of standard geneNorm and NormFinder algorithms from 6 possible genes included on Exiqon microRNA Ready-to-Use PCR Panels. Only miRNA with average Ct (threshold cycle) lower than 35 in one group were statically evaluated. Statistical differences between the levels of analyzed miRNAs in *P. acnes* positive and *P. acnes* negative cases were evaluated by non-parametric Mann-Whitney test with Benjamimi-Hochberg correction for multiple hypothesis testing. Individual microRNA expression levels were quantified relatively to the average of two reference genes (RNU38B and RNU48). Samples with Ct(RNU38B)>34 and/or Ct(RNU48)>31 were excluded from the study. Diagnostic MicroRNA Score (DMS) formula was developed based on a linear combination of the miR-29a-3p and miR-574-3p expression levels. Optimal cut-off value to discriminate *P. acnes* positive and *P. acnes* negative cases was obtained through ROC analysis. Calculations were performed using GraphPad Prism version 7.00 (GraphPad Software, San Diego, CA, USA). P-values of less than 0.05 were considered statistically significant.

TABLE 2: miRNAs

|  |  |  |
|---|---|---|
|  | hsa-miR-369-5p | hsa-miR-302c-3p |
| hsa-miR-7-5p | hsa-miR-425-5p | hsa-miR-625-3p |
| hsa-miR-217 | hsa-miR-590-5p | hsa-miR-339-5p |
| hsa-miR-337-5p | hsa-miR-760 | hsa-miR-873-5p |
| hsa-miR-328-3p | hsa-miR-574-3p | hsa-miR-323a-3p |
| hsa-miR-374b-3p | hsa-miR-130b-3p | hsa-miR-181d-5p |
| hsa-miR-143-3p | hsa-miR-30c-5p | hsa-miR-125a-5p |
| hsa-miR-623 | hsa-miR-133b | hsa-miR-129-5p |
| hsa-miR-520c-3p | hsa-miR-524-5p | hsa-miR-492 |
| hsa-miR-557 | hsa-miR-23a-3p | hsa-miR-20a-5p |
| hsa-miR-218-5p | hsa-miR-193b-3p | hsa-miR-374b-5p |
| hsa-miR-136-5p | hsa-miR-501-5p | hsa-miR-302d-3p |
| hsa-miR-127-5p | hsa-miR-518c-5p | hsa-miR-346 |
| hsa-miR-140-5p | hsa-miR-130a-3p | hsa-miR-151a-3p |
| hsa-miR-31-3p | hsa-miR-933 | hsa-miR-493-3p |
| hsa-miR-20b-3p | hsa-miR-379-5p | hsa-miR-122-5p |
| hsa-miR-325 | hsa-miR-452-5p | hsa-miR-99a-3p |
| hsa-miR-509-3-5p | hsa-miR-589-5p | hsa-miR-361-5p |
| hsa-miR-210-3p | hsa-miR-141-3p | hsa-miR-202-3p |
| hsa-miR-199b-5p | hsa-miR-342-3p | hsa-miR-125b-5p |
| hsa-miR-194-5p | hsa-miR-668-3p | hsa-miR-503-5p |
| hsa-let-7g-5p | hsa-miR-934 | hsa-miR-204-5p |
| hsa-miR-203a | hsa-miR-101-3p | hsa-miR-30d-5p |
| hsa-miR-181a-3p | hsa-miR-539-5p | hsa-miR-301a-3p |
| hsa-miR-137 | hsa-miR-331-3p | hsa-miR-362-5p |
| hsa-miR-551b-3p | hsa-miR-499a-5p | hsa-miR-30b-3p |
| hsa-miR-524-3p | hsa-miR-196a-5p | hsa-miR-654-5p |
| hsa-miR-486-5p | hsa-miR-888-5p | hsa-miR-545-3p |
| hsa-miR-329-3p | hsa-miR-330-3p | hsa-miR-29b-2-5p |
| hsa-miR-487b-3p | hsa-miR-570-3p | hsa-miR-491-5p |
| cel-miR-39-3p | hsa-miR-518c-3p | hsa-miR-92b-3p |
| hsa-miR-138-5p | hsa-miR-200a-3p | hsa-miR-665 |
| hsa-miR-191-5p | hsa-miR-188-5p | hsa-miR-506-3p |
| mmu-miR-378a-3p | hsa-miR-26a-5p | hsa-miR-363-3p |
| hsa-miR-103a-3p | hsa-miR-99b-5p | hsa-miR-132-3p |
| hsa-miR-890 | hsa-miR-431-5p | hsa-miR-651-5p |

(continued)

| | | |
|---|---|---|
| hsa-miR-423-5p | hsa-miR-23b-3p | hsa-miR-628-3p |
| hsa-miR-221-3p | hsa-miR-367-3p | hsa-miR-432-5p |
| hsa-miR-301b | hsa-miR-505-3p | hsa-miR-154-3p |
| hsa-miR-550a-5p | hsa-miR-18a-5p | hsa-miR-27a-3p |
| hsa-miR-532-5p | hsa-miR-92a-3p | hsa-miR-376c-3p |
| hsa-miR-99a-5p | hsa-miR-500a-5p | hsa-miR-940 |
| hsa-miR-16-5p | hsa-miR-887-3p | hsa-miR-22-5p |
| hsa-miR-98-5p | hsa-miR-491-3p | hsa-miR-224-5p |
| hsa-miR-185-5p | hsa-miR-423-3p | hsa-miR-885-5p |
| hsa-miR-25-3p | hsa-miR-126-3p | hsa-miR-320a |
| hsa-miR-765 | hsa-miR-421 | hsa-miR-18b-5p |
| hsa-miR-24-3p | hsa-miR-376b-3p | hsa-miR-187-3p |
| hsa-miR-516b-5p | hsa-miR-129-2-3p | hsa-miR-512-5p |
| hsa-miR-302c-5p | hsa-miR-26b-5p | hsa-miR-449a |
| hsa-miR-548b-3p | hsa-miR-214-3p | hsa-miR-498 |
| hsa-miR-186-5p | hsa-miR-32-5p | hsa-miR-148b-3p |
| hsa-miR-199a-5p | hsa-miR-324-3p | hsa-miR-127-3p |
| hsa-miR-155-5p | hsa-miR-488-3p | hsa-miR-598-3p |
| hsa-miR-107 | hsa-miR-371a-5p | hsa-miR-96-5p |
| hsa-miR-302b-3p | hsa-miR-455-5p | hsa-let-7d-5p |
| hsa-miR-662 | hsa-miR-891a-5p | hsa-miR-135b-5p |
| hsa-miR-519d-3p | hsa-miR-549a | hsa-miR-495-3p |
| hsa-miR-485-3p | hsa-miR-205-5p | hsa-miR-299-5p |
| hsa-miR-200b-3p | hsa-miR-518b | hsa-miR-34c-3p |
| hsa-miR-337-3p | hsa-miR-19a-3p | hsa-miR-596 |
| hsa-miR-494-3p | hsa-miR-150-5p | hsa-miR-744-5p |
| hsa-miR-371a-3p | hsa-miR-15a-5p | hsa-miR-145-5p |
| hsa-miR-637 | hsa-let-7d-3p | hsa-miR-622 |
| hsa-miR-144-3p | hsa-miR-608 | hsa-miR-516a-5p |
| hsa-miR-16-1-3p | hsa-miR-671-5p | hsa-let-7a-5p |
| hsa-miR-631 | hsa-miR-497-5p | hsa-miR-96-3p |
| hsa-miR-34c-5p | hsa-miR-877-5p | hsa-miR-185-3p |
| hsa-miR-211-Sp | hsa-miR-187-5p | hsa-miR-615-3p |
| hsa-miR-454-3p | hsa-miR-10b-5p | hsa-miR-128-3p |
| hsa-let-7f-5p | hsa-let-7i-5p | hsa-miR-766-3p |
| hsa-miR-30e-5p | hsa-miR-202-5p | hsa-miR-206 |
| hsa-miR-34a-5p | hsa-miR-652-3p | hsa-miR-298 |
| hsa-miR-663a | hsa-miR-126-5p | hsa-miR-193a-5p |
| hsa-miR-518e-3p | hsa-miR-30e-3p | hsa-miR-449b-5p |
| hsa-miR-29b-3p | hsa-miR-181c-5p | hsa-miR-520d-5p |
| hsa-miR-658 | hsa-miR-9-3p | hsa-miR-192-5p |
| hsa-miR-572 | hsa-miR-548c-3p | hsa-miR-29a-3p |
| hsa-miR-802 | hsa-miR-152-3p | hsa-miR-18a-3p |
| hsa-miR-521 | hsa-miR-93-5p | hsa-miR-383-5p |
| hsa-miR-433-3p | hsa-miR-365a-3p | hsa-miR-9-5p |
| hsa-miR-660-5p | hsa-miR-29c-3p | hsa-miR-142-5p |
| hsa-let-7c-5p | hsa-miR-372-3p | hsa-miR-363-5p |
| hsa-miR-28-5p | hsa-miR-133a-3p | hsa-miR-147b |
| hsa-miR-324-5p | hsa-miR-124-3p | hsa-miR-197-3p |
| hsa-miR-219a-5p | hsa-miR-190a-5p | hsa-miR-597-5p |
| hsa-miR-19b-3p | hsa-miR-302a-3p | hsa-miR-326 |

(continued)

| | | |
|---|---|---|
| hsa-miR-526b-5p | hsa-miR-595 | hsa-miR-15b-5p |
| hsa-miR-215-5p | hsa-miR-602 | hsa-miR-105-5p |
| hsa-miR-30b-5p | hsa-miR-223-3p | hsa-miR-196b-5p |
| hsa-miR-184 | hsa-miR-627-5p | hsa-miR-296-5p |
| hsa-miR-422a | hsa-miR-34b-3p | hsa-miR-20b-5p |
| hsa-miR-199a-3p | hsa-miR-410-3p | hsa-miR-147a |
| hsa-miR-335-5p | hsa-miR-17-5p | hsa-miR-198 |
| hsa-miR-519a-3p | hsa-miR-376a-3p | hsa-miR-375 |
| hsa-miR-21-5p | hsa-miR-514a-3p | hsa-miR-517a-3p |
| hsa-miR-361-3p | hsa-miR-216b-5p | hsa-miR-25-5p |
| hsa-miR-21-3p | hsa-miR-106b-5p | hsa-miR-922 |
| hsa-miR-373-3p | hsa-miR-22-3p | hsa-miR-124-5p |
| hsa-miR-518f-3p | hsa-miR-510-5p | hsa-miR-1264 |
| hsa-miR-222-3p | hsa-miR-212-3p | hsa-miR-504-5p |
| hsa-miR-617 | hsa-miR-525-5p | hsa-miR-138-1-3p |
| hsa-miR-154-5p | hsa-miR-542-5p | hsa-miR-502-3p |
| hsa-miR-708-5p | hsa-miR-576-3p | hsa-miR-490-5p |
| hsa-let-7b-5p | hsa-miR-583 | hsa-miR-567 |
| hsa-miR-95-3p | hsa-miR-483-3p | hsa-miR-18b-3p |
| hsa-miR-517c-3p | hsa-miR-582-5p | hsa-miR-125a-3p |
| hsa-miR-151a-5p | hsa-miR-183-5p | hsa-miR-653-5p |
| hsa-miR-502-5p | hsa-miR-33b-5p | hsa-miR-891b |
| hsa-miR-345-5p | hsa-miR-193a-3p | hsa-miR-144-5p |
| hsa-miR-509-3p | hsa-miR-153-3p | hsa-miR-1538 |
| hsa-miR-134-5p | hsa-let-7e-5p | hsa-miR-384 |
| hsa-miR-382-5p | hsa-miR-409-3p | hsa-miR-196b-3p |
| hsa-miR-490-3p | hsa-miR-100-5p | hsa-miR-649 |
| hsa-miR-200c-3p | hsa-miR-629-5p | hsa-miR-143-5p |
| hsa-miR-30a-5p | hsa-miR-484 | hsa-miR-1207-5p |
| hsa-miR-181b-5p | hsa-miR-429 | hsa-miR-943 |
| hsa-miR-33a-5p | hsa-miR-30c-2-3p | hsa-miR-675-3p |
| hsa-miR-195-5p | hsa-miR-518a-3p | hsa-miR-200b-5p |
| hsa-miR-874-3p | hsa-miR-340-5p | hsa-miR-519e-5p |
| hsa-miR-135a-5p | hsa-miR-508-3p | hsa-miR-942-5p |
| hsa-miR-26a-2-3p | hsa-miR-381-3p | hsa-miR-450b-3p |
| hsa-miR-146b-5p | hsa-miR-148a-3p | hsa-miR-553 |
| hsa-miR-412-3p | hsa-miR-146a-5p | hsa-miR-605-5p |
| hsa-miR-1 | hsa-miR-139-5p | hsa-miR-24-2-5p |
| hsa-miR-299-3p | hsa-miR-373-5p | hsa-miR-23a-5p |
| hsa-miR-142-3p | hsa-miR-149-5p | hsa-miR-27b-5p |
| hsa-miR-338-3p | hsa-miR-642a-5p | hsa-miR-759 |
| hsa-miR-584-5p | hsa-miR-31-5p | hsa-miR-770-5p |
| hsa-miR-377-3p | hsa-miR-451a | hsa-miR-585-3p |
| hsa-miR-216a-5p | hsa-miR-620 | hsa-miR-376a-5p |
| hsa-miR-424-5p | hsa-miR-27b-3p | hsa-miR-507 |
| hsa-miR-921 | hsa-miR-523-3p | hsa-miR-520b |
| hsa-miR-513a-5p | hsa-miR-374a-5p | hsa-miR-302f |
| hsa-miR-140-3p | hsa-miR-92a-1-5p | hsa-miR-28-3p |
| hsa-miR-181a-5p | hsa-miR-219a-1-3p | hsa-miR-875-5p |
| hsa-miR-10a-5p | hsa-miR-1913 | hsa-miR-219a-2-3p |
| hsa-miR-106a-5p | hsa-miR-1245a | hsa-miR-1183 |

(continued)

| | | |
|---|---|---|
| hsa-miR-182-5p | hsa-miR-522-3p | hsa-miR-758-3p |
| hsa-miR-370-3p | hsa-miR-571 | hsa-miR-1244 |
| hsa-miR-576-5p | hsa-miR-323a-5p | hsa-miR-566 |
| hsa-miR-425-3p | hsa-miR-592 | hsa-miR-1256 |
| hsa-miR-450a-5p | hsa-miR-487a-3p | hsa-miR-516a-3p |
| hsa-miR-411-5p | hsa-miR-1249 | hsa-miR-548c-5p |
| | hsa-miR-621 | hsa-miR-1260a |
| hsa-miR-496 | hsa-miR-556-5p | hsa-miR-182-3p |
| hsa-miR-876-3p | hsa-miR-1267 | hsa-miR-365b-5p |
| hsa-miR-532-3p | hsa-miR-141-5p | hsa-miR-508-5p |
| hsa-miR-654-3p | hsa-miR-1269a | hsa-miR-671-3p |
| hsa-miR-659-3p | hsa-miR-501-3p | hsa-miR-941 |
| hsa-miR-13 5b-3p | hsa-miR-15b-3p | hsa-miR-23b-5p |
| hsa-miR-641 | hsa-miR-146b-3p | hsa-miR-591 |
| hsa-miR-2113 | hsa-miR-222-5p | hsa-miR-26b-3p |
| hsa-miR-1254 | hsa-miR-601 | hsa-miR-519b-3p |
| hsa-miR-661 | hsa-miR-924 | hsa-miR-30d-3p |
| hsa-miR-892a | hsa-miR-29a-5p | hsa-miR-518d-5p |
| hsa-miR-10b-3p | hsa-let-7a-2-3p | hsa-miR-212-5p |
| hsa-miR-122-3p | hsa-miR-520f-3p | hsa-miR-520e |
| hsa-miR-100-3p | hsa-miR-101-5p | hsa-miR-646 |
| hsa-miR-769-3p | hsa-miR-520a-3p | hsa-miR-519e-3p |
| hsa-miR-300 | hsa-miR-548m | hsa-miR-626 |
| hsa-miR-518e-5p | hsa-miR-517-5p | hsa-miR-26a-1-3p |
| hsa-miR-489-3p | hsa-miR-448 | hsa-miR-190b |
| hsa-miR-937-3p | hsa-miR-1296-5p | hsa-miR-1471 |
| hsa-miR-381-5p | hsa-miR-1537-3p | hsa-miR-5481 |
| hsa-miR-640 | hsa-miR-920 | hsa-miR-586 |
| hsa-miR-148b-5p | hsa-miR-1247-5p | hsa-miR-103b |
| hsa-miR-29c-5p | hsa-miR-19b-2-5p | hsa-miR-488-5p |
| hsa-miR-499a-3p | hsa-miR-558 | hsa-miR-129-1-3p |
| hsa-let-7f-1-3p | hsa-miR-106b-3p | hsa-miR-192-3p |
| hsa-miR-382-3p | hsa-miR-1258 | hsa-miR-632 |
| hsa-miR-609 | hsa-miR-619-3p | hsa-miR-181a-2-3p |
| hsa-miR-10a-3p | hsa-miR-208a-3p | hsa-miR-1909-3p |
| hsa-miR-106a-3p | hsa-miR-17-3p | hsa-miR-573 |
| hsa-let-7e-3p | hsa-miR-136-3p | hsa-miR-302d-5p |
| hsa-miR-580-3p | hsa-miR-877-3p | hsa-miR-194-3p |
| hsa-miR-761 | hsa-miR-935 | hsa-miR-302b-5p |
| hsa-miR-643 | hsa-miR-224-3p | hsa-miR-551b-5p |
| hsa-miR-618 | hsa-miR-624-3p | hsa-miR-635 |
| hsa-miR-221-5p | hsa-miR-767-5p | hsa-miR-518d-3p |
| hsa-miR-513b-5p | hsa-miR-559 | hsa-miR-569 |
| hsa-miR-411-3p | hsa-miR-449b-3p | hsa-miR-125b-1-3p |
| hsa-miR-19a-5p | hsa-miR-205-3p | hsa-miR-218-2-3p |
| hsa-miR-338-5p | hsa-miR-604 | hsa-miR-519c-3p |
| hsa-miR-1914-3p | hsa-miR-130b-5p | hsa-miR-554 |
| hsa-miR-323b-5p | hsa-miR-149-3p | hsa-miR-938 |
| hsa-miR-548i | hsa-miR-1271-5p | hsa-miR-1243 |
| hsa-miR-541-3p | hsa-miR-520h | hsa-miR-708-3p |
| hsa-miR-1272 | hsa-miR-769-5p | hsa-miR-1185-5p |

(continued)

| | | |
|---|---|---|
| hsa-miR-1205 | hsa-miR-612 | hsa-miR-512-3p |
| hsa-miR-544a | hsa-miR-1237-3p | hsa-miR-587 |
| hsa-miR-431-3p | hsa-miR-1908-5p | hsa-miR-603 |
| hsa-miR-1184 | hsa-miR-1255b-5p | hsa-miR-1200 |
| hsa-miR-20a-3p | hsa-miR-330-5p | hsa-miR-1911-5p |
| hsa-miR-588 | hsa-miR-1238-3p | hsa-miR-33b-3p |
| hsa-miR-455-3p | hsa-miR-188-3p | hsa-miR-223-5p |
| hsa-miR-582-3p | hsa-miR-589-3p | hsa-miR-34b-5p |
| hsa-miR-409-5p | hsa-miR-125b-2-3p | hsa-miR-888-3p |
| hsa-miR-452-3p | hsa-miR-16-2-3p | hsa-miR-424-3p |
| hsa-miR-19b-1-5p | hsa-miR-515-5p | hsa-miR-339-3p |
| hsa-miR-610 | hsa-miR-340-3p | hsa-miR-380-5p |
| hsa-miR-511-5p | hsa-miR-513a-3p | hsa-miR-647 |
| hsa-miR-200c-5p | hsa-miR-34a-3p | hsa-miR-518f-5p |
| hsa-let-7a-3p | hsa-miR-342-5p | hsa-miR-92b-5p |
| hsa-miR-135a-3p | hsa-miR-639 | hsa-miR-551a |
| hsa-miR-520a-5p | hsa-let-7i-3p | hsa-miR-146a-3p |
| hsa-miR-1468-5p | hsa-miR-543 | hsa-miR-218-1-3p |
| hsa-miR-628-5p | hsa-miR-645 | hsa-miR-593-5p |
| hsa-miR-552-3p | hsa-miR-548d-5p | hsa-miR-561-3p |
| hsa-miR-145-3p | hsa-miR-33a-3p | hsa-miR-767-3p |
| hsa-miR-378a-5p | hsa-miR-664a-3p | hsa-miR-526b-3p |
| hsa-miR-7-1-3p | hsa-miR-379-3p | hsa-miR-24-1-5p |
| hsa-miR-181c-3p | hsa-miR-556-3p | hsa-let-7b-3p |
| hsa-miR-195-3p | hsa-miR-614 | hsa-miR-193b-5p |
| hsa-miR-578 | hsa-miR-616-5p | hsa-miR-335-3p |
| hsa-miR-505-5p | hsa-miR-93-3p | hsa-miR-541-5p |
| hsa-miR-875-3p | hsa-miR-1972 | hsa-miR-30c-1-3p |
| hsa-miR-450b-5p | hsa-miR-616-3p | hsa-miR-629-3p |
| hsa-miR-876-5p | hsa-miR-369-3p | hsa-miR-377-5p |
| hsa-miR-362-3p | hsa-miR-2110 | hsa-miR-630 |
| hsa-miR-624-5p | hsa-miR-548a-3p | hsa-miR-548d-3p |
| hsa-miR-27a-5p | hsa-miR-634 | hsa-miR-885-3p |
| hsa-miR-744-3p | hsa-miR-320c | hsa-miR-320d |
| hsa-miR-139-3p | hsa-miR-636 | hsa-miR-2053 |
| hsa-miR-138-2-3p | hsa-miR-606 | hsa-miR-675-5p |
| hsa-miR-655-3p | hsa-miR-208b-3p | hsa-miR-1252-5p |
| hsa-miR-99b-3p | hsa-miR-367-5p | hsa-miR-548e-3p |
| hsa-miR-581 | hsa-miR-520d-3p | hsa-miR-1914-5p |
| hsa-miR-191-3p | hsa-miR-1265 | hsa-miR-513c-5p |
| hsa-miR-32-3p | hsa-miR-1203 | hsa-miR-331-5p |
| hsa-miR-1204 | hsa-miR-548k | hsa-miR-1182 |
| hsa-miR-548j-5p | hsa-miR-548a-5p | hsa-miR-611 |
| hsa-miR-555 | hsa-miR-1253 | hsa-miR-1181 |
| hsa-miR-1224-3p | hsa-miR-615-5p | hsa-miR-638 |
| hsa-miR-1539 | hsa-miR-607 | hsa-miR-515-3p |
| hsa-miR-663b | hsa-miR-1208 | hsa-miR-650 |
| hsa-miR-1248 | hsa-miR-302e | hsa-miR-1178-3p |
| hsa-miR-889-3p | hsa-miR-1206 | hsa-miR-600 |
| hsa-miR-1227-3p | hsa-miR-1270 | hsa-miR-599 |
| hsa-miR-548h-5p | hsa-miR-525-3p | hsa-miR-520g-3p |

(continued)

| | | |
|---|---|---|
| hsa-miR-564 | hsa-miR-7-2-3p | hsa-miR-183-3p |
| hsa-miR-132-5p | hsa-miR-550a-3p | hsa-miR-1179 |
| hsa-miR-577 | hsa-miR-380-3p | hsa-miR-562 |
| hsa-miR-1911-3p | hsa-miR-593-3p | hsa-miR-579-3p |
| hsa-let-7f-2-3p | hsa-miR-1912 | hsa-miR-590-3p |
| hsa-miR-155-3p | hsa-miR-493-5p | hsa-miR-130a-5p |
| hsa-miR-105-3p | hsa-miR-432-3p | hsa-miR-563 |
| hsa-miR-486-3p | hsa-miR-454-5p | hsa-miR-200a-5p |
| hsa-miR-320b | hsa-miR-936 | hsa-miR-483-5p |
| hsa-miR-296-3p | hsa-miR-30a-3p | hsa-miR-15a-3p |
| | hsa-let-7g-3p | hsa-miR-944 |
| | hsa-miR-214-5p | hsa-miR-92a-2-5p |
| | | hsa-miR-548 n |

TABLE 6: Application of DMS in cases with non-abundant *P. acnes* by culture and various levels of *P. acnes* genomes by PCR (inconclusive by standard methods)

| ID | P. acnes culture [CFU/ml] | Culture -result | PCR [P. acnes genomes / 500ng DNA] | PCR - result * | DMS value | DMS- result | All results | P. acnes diagnosis after DMS correction |
|---|---|---|---|---|---|---|---|---|
| 1 | 800 | + | 194 | + | -0.31 | + | +/+/+ | POSITIVE |
| 2 | 800 | + | 0 | - | 1.20 | - | +/-/- | FALSE POSITIVE |
| 3 | 800 | + | 1301 | ++ | 2.88 | - | +/++/- | FALSE POSITIVE |
| 4 | 700 | + | 618 | ++ | -2.41 | + | +/++/+ | POSITIVE |
| 5 | 600 | + | 392 | + | -0.43 | + | +/+/+ | POSITIVE |
| 6 | 600 | + | 0 | - | -3.62 | + | +/-/+ | POSITIVE |
| 7 | 600 | + | 193 | + | -0.49 | + | +/+/+ | POSITIVE |
| 8 | 500 | + | 1155 | ++ | -39.68 | + | +/++/+ | POSITIVE |
| 9 | 500 | + | 348 | + | -4.83 | + | +/+/+ | POSITIVE |
| 10 | 500 | + | 2361 | ++ | 0.36 | - | +/++/- | FALSE POSITIVE |
| 11 | 500 | + | 0 | - | 3.42 | - | +/-/- | FALSE POSITIVE |
| 12 | 500 | + | 17 | + | 0.37 | - | +/+/- | FALSE POSITIVE |
| 13 | 300 | + | 294 | + | -1.84 | + | +/+/+ | POSITIVE |
| 14 | 300 | + | 0 | - | 6.26 | - | +/-/- | FALSE POSITIVE |
| 15 | 300 | + | 0 | - | -0.93 | + | +/-/+ | POSITIVE |
| 16 | 300 | + | 0 | - | 6.05 | - | +/-/- | FALSE POSITIVE |
| 17 | 300 | + | 0 | - | -0.44 | + | +/-/+ | POSITIVE |
| 18 | 200 | + | 271 | + | 0.07 | + | +/+/+ | POSITIVE |
| 19 | 200 | + | 191 | + | -2.2 | + | +/+/+ | POSITIVE |
| 20 | 200 | + | 64 | + | -2.86 | + | +/+/+ | POSITIVE |
| 21 | 200 | + | 0 | - | 6.57 | - | +/-/- | FALSE POSITIVE |
| 22 | 200 | + | 0 | - | 2.52 | - | +/-/- | FALSE POSITIVE |
| 23 | 200 | + | 0 | - | 4.18 | - | +/-/- | FALSE POSITIVE |

(continued)

| ID | P. acnes culture [CFU/ml] | Culture -result | PCR [P. acnes genomes / 500ng DNA] | PCR - result * | DMS value | DMS- result | All results | P. acnes diagnosis after DMS correction |
|---|---|---|---|---|---|---|---|---|
| 24 | 200 | + | 0 | - | -1.63 | + | +/-/+ | POSITIVE |
| 25 | 200 | + | 0 | - | 2.41 | - | +/-/- | FALSE POSITIVE |
| 26 | 200 | + | 0 | - | 5.24 | - | +/-/- | FALSE POSITIVE |
| 27 | 200 | + | 48 | + | 11.97 | - | +/+/- | FALSE POSITIVE |
| 28 | 100 | + | 0 | - | 2.04 | - | +/-/- | FALSE POSITIVE |
| 29 | 100 | + | 0 | - | -1.08 | + | +/-/+ | POSITIVE |
| 30 | 100 | + | 213 | + | 1.1 | - | +/+/- | FALSE POSITIVE |
| 31 | 100 | + | 0 | - | -0.24 | - | +/-/- | FALSE POSITIVE |
| 32 | 100 | + | 0 | - | 5.73 | - | +/-/- | FALSE POSITIVE |
| 33 | 100 | + | 0 | - | 5.92 | - | +/-/- | FALSE POSITIVE |
| 34 | 100 | + | 249 | + | 2.42 | - | +/+/- | FALSE POSITIVE |
| 35 | 100 | + | 0 | - | 1.64 | - | +/-/- | FALSE POSITIVE |
| 36 | 100 | + | 80 | + | 4.97 | - | +/+/- | FALSE POSITIVE |
| 37 | 100 | + | 0 | - | 2.55 | - | +/-/- | FALSE POSITIVE |
| 38 | 100 | + | 0 | - | 0.12 | - | +/-/- | FALSE POSITIVE |
| 39 | 100 | + | 0 | - | -0.35 | + | +/-/+ | POSITIVE |
| 40 | 0 | - | 1500 | ++ | 12.34 | - | -/++/- | FALSE PCR POSITIVE |
| 41 | 0 | - | 995 | ++ | 3.84 | - | -/++/- | FALSE PCR POSITIVE |
| 42 | 0 | - | 555 | ++ | 8.39 | - | -/++/- | FALSE PCR POSITIVE |
| 43 | 0 | - | 572 | ++ | 4.78 | - | -/++/- | FALSE PCR POSITIVE |
| 44 | 0 | - | 2046 | ++ | -2.26 | + | -/++/+ | FALSE NEGATIVE CULTURE |

*++ means CFU/ml > 103; ** ++ means number of P. acnes genomes higher than 500

**Claims**

1. A method for detecting a low-virulence infection with *Propionibacterium acnes* in a patient, comprising:

   testing for the presence or level of *Propionibacterium acnes* in a patient's intervertebral disc sample by one or more of PCR, culture, immunohistochemistry, spectroscopy, in situ hybridization, or metagenomics analysis, *Propionibacterium acnes* being causative of low-virulence infections,
   determining a host cell diagnostic microRNA score (DMS) from said sample;
   evaluating the microRNA profile for a microRNA signature indicative of a low-virulence infection to discriminate false positives and/or false negatives from true low-virulence infections; wherein the microRNA signature is trained with microRNA signatures from infected clinical samples or samples that test positive for the presence or abundance of *Propionibacterium acnes,* and uninfected clinical samples or samples that test negative for the presence or abundance of *Propionibacterium acnes.*

2. The method of claim 1, wherein the patient has symptoms of an inflammatory condition, wherein the symptoms of an inflammatory condition are from chronic lower back pain (CLBP)

3. The method of any one of claims 1 or 2, wherein the patient has a history of chronic low back pain (CLBP) and/or failed back surgery; or the patient has a history of pseudoarthrosis.

4. The method of any one of claims 1 to 3, with a sample obtained at the time of surgery, or the sample obtained by biopsy prior to surgery.

5. The method of any of claim 1-4, wherein the RNA signature includes the relative expression of two or three RNAs.

6. The method of claim 5, wherein the RNAs are miRNAs selected from Table 4, Table 5, or Figure 8.

7. The method of claim 5 or 6, wherein the RNA signature includes the relative expression level of one or both of miR-29a-3p and miR-574-3p.


**Patentansprüche**

1. Ein Verfahren zum Nachweis einer schwach-virulenten Infektion mit *Propionibacterium acnes* bei einem Patienten, umfassend:

   Testen auf das Vorhandensein oder das Level von *Propionibacterium acnes* in einer Bandscheibenprobe eines Patienten durch eines oder mehrere der folgenden: PCR, Kultur, Immunhistochemie, Spektroskopie, In-situ-Hybridisierung oder Metagenomics-Analyse, wobei *Propionibacterium acnes* für schwach-virulente Infektionen ursächlich ist,
   Bestimmen eines wirtszelldiagnostischen microRNA-Scores (DMS) aus jener Probe;
   Auswerten des microRNA-Profils auf eine microRNA-Signatur, die auf eine schwach-virulente Infektion hinweist, um falsch positive und/oder falsch negative von echten schwach-virulenten Infektionen zu unterscheiden; wobei die microRNA-Signatur mit microRNA-Signaturen aus infizierten klinischen Proben oder Proben, die positiv auf die Präsenz oder die Abundanz von *Propionibacterium acnes* getestet wurden, und nicht infizierten klinischen Proben oder Proben, die negativ auf die Präsenz oder die Abundanz von *Propionibacterium acnes* getestet wurden, trainiert wird.

2. Das Verfahren nach Anspruch 1, wobei der Patient Symptome eines entzündlichen Zustands hat, wobei die Symptome eines entzündlichen Zustands von chronischen unteren Rückenschmerzen (CLBP) herrühren.

3. Das Verfahren nach einem der Ansprüche 1 oder 2, wobei der Patient eine Vorgeschichte von chronischen unteren Rückenschmerzen (CLBP) und/oder misslungenen Rückenoperationen hat; oder der Patient eine Vorgeschichte von Pseudoarthrose hat.

4. Das Verfahren nach einem der Ansprüche 1 bis 3, wobei die Probe zum Zeitpunkt der Operation entnommen wird, oder die Probe durch Biopsie vor der Operation entnommen wird.

5. Das Verfahren nach einem der Ansprüche 1 bis 4, wobei die RNA-Signatur die relative Expression von zwei oder drei RNAs umfasst.

6. Das Verfahren nach Anspruch 5, wobei die RNAs miRNAs sind, ausgewählt aus Tabelle 4, Tabelle 5 oder Figur 8.

7. Das Verfahren nach Anspruch 5 oder 6, wobei die RNA-Signatur das relative Expressionslevel von einer oder beiden von miR-29a-3p und miR-574-3p umfasst.


**Revendications**

1. Procédé de détection d'une infection à faible virulence avec *Propionibacterium acnes* chez un patient, comprenant :

   un test de la présence ou du niveau de *Propionibacterium acnes* dans un échantillon de disque intervertébral du patient par une ou plusieurs parmi une PCR, une culture, une immunohistochimie, une spectroscopie, une hybridation in situ ou une analyse métagénomique, *Propionibacterium acnes* provoquant des infections à faible virulence,

la détermination d'un score diagnostique de micro-ARN (DMS) de cellule hôte à partir dudit échantillon ;

l'évaluation du profil de micro-ARN pour une signature de micro-ARN indiquant une infection à faible virulence pour distinguer les faux positifs et/ou les faux négatifs de vraies infections à faible virulence ; dans lequel la signature de micro-ARN est entraînée avec des signatures de micro-ARN provenant d'échantillons cliniques infectés ou d'échantillons qui sont testés positifs à la présence ou l'abondance de *Propionibacterium acnes,* et d'échantillons cliniques non infectés ou d'échantillons qui sont testés négatifs à la présence ou l'abondance de *Propionibacterium acnes.*

2. Procédé selon la revendication 1, dans lequel le patient présente des symptômes d'un état inflammatoire, dans lequel les symptômes d'un état inflammatoire proviennent d'une lombalgie chronique (CLBP).

3. Procédé selon l'une quelconque des revendications 1 ou 2, dans lequel le patient présente des antécédents de lombalgie chronique (CLBP) et/ou d'intervention chirurgicale du dos ayant échoué ; ou le patient présente des antécédents de pseudarthrose.

4. Procédé selon l'une quelconque des revendications 1 à 3, avec un échantillon obtenu au moment d'une intervention chirurgicale, ou l'échantillon obtenu par biopsie avant une intervention chirurgicale.

5. Procédé selon l'une quelconque des revendications 1 à 4, dans lequel la signature d'ARN inclut l'expression relative de deux ou trois ARN.

6. Procédé selon la revendication 5, dans lequel les ARN sont des miARN sélectionnés dans le tableau 4, dans le tableau 5, ou sur la figure 8.

7. Procédé selon la revendication 5 ou 6, dans lequel la signature d'ARN inclut le niveau d'expression relatif d'un ou des deux parmi miR-29a-3p et miR-574-3p.

FIGURE 1

Normal disc

Herniated disc

Nerve root

Decreased height of vertebral body

Bone spurs

Degenerated disc

Lumbar spine

Sacrum

*Image Courtesy of ReedGroup's MDGuidelines*

Normal Disc

Degenerated Disc. Shows less water content (blacker) on MRI.

Herniated Disc. This disc is also degenerated.

© Thomas Deloner 2007 / Alamy Stock Photo

FIGURE 2

Scheduled Intervertebral Disc Surgery → Intervertebral Disc Sample → Diagnostic Tests PCR & miRNA → Low Virulence Infection Diagnosis → Antibiotic, local antiseptic or surgical treatments as individually required

# FIGURE 3

Dudli, Stefan, "Post-Traumatic Intervertebral Disc Degeneration – An In Vitro Study on the Etiopathogenesis", Ph.D. Thesis, Universität Zürich, 2013.

FIGURE 4

miR-574-3p

miR-29a-3p

miR-497-5p

miR-29c-3p

miR-99b-5p

FIGURE 5

A

**DMS (miR-29a-3p / miR-574-3p)**

B

**DMS (miR-29a-3p / miR-574-3p)**

AUC = 0.9833
P < 0.0001

EP 3 325 624 B1

FIGURE 6

A    DMS (miR-29a-3p / miR-574-3p)

B    DMS (miR-29a-3p / miR-574-3p)

EP 3 325 624 B1

FIGURE 7

DMS (miR-29a-3p / miR-574-3p)

EP 3 325 624 B1

FIGURE 8

**hsa-miR-125b-2-3p**

miRBase accession number: MIMAT0004603

UCACAAGUCAGGCUCUUGGGAC (SEQ ID NO:21)

**hsa-miR-99a-5p**

miRBase accession number: MIMAT0000097

AACCCGUAGAUCCGAUCUUGUG (SEQ ID NO:22)

**hsa-miR-29a-3p**

miRBase accession number: MIMAT0000086

UAGCACCAUCUGAAAUCGGUUA (SEQ ID NO:23)

**hsa-miR-99b-5p**

miRBase accession number: MIMAT0000689

CACCCGUAGAACCGACCUUGCG (SEQ ID NO:24)

**hsa-miR-125b-5p**

miRBase accession number: MIMAT0000423

UCCCUGAGACCCUAACUUGUGA (SEQ ID NO:25)

**hsa-miR-28-3p**

miRBase accession number: MIMAT0004502

CACUAGAUUGUGAGCUCCUGGA (SEQ ID NO:26)

FIGURE 8 (continued)

**hsa-miR-92b-3p**

miRBase accession number: MIMAT0003218

UAUUGCACUCGUCCCGGCCUCC (SEQ ID NO:27)

**hsa-miR-29c-3p**

miRBase accession number: MIMAT0000681

UAGCACCAUUUGAAAUCGGUUA (SEQ ID NO:28)

**hsa-miR-497-5p**

miRBase accession number: MIMAT0002820

CAGCAGCACACUGUGGUUUGU (SEQ ID NO:29)

**hsa-miR-28-5p**

miRBase accession number: MIMAT0000085

AAGGAGCUCACAGUCUAUUGAG (SEQ ID NO:30)

**hsa-miR-125a-5p**

miRBase accession number: MIMAT0000443

UCCCUGAGACCCUUUAACCUGUGA (SEQ ID NO:31)

**hsa-miR-140-3p**

miRBase accession number: MIMAT0004597

UACCACAGGGUAGAACCACGG (SEQ ID NO:32)

FIGURE 8 (continued)


**hsa-miR-574-3p**

miRBase accession number: MIMAT0003239

CACGCUCAUGCACACACCCACA (SEQ ID NO:33)


**hsa-miR-16-2-3p**

miRBase accession number: MIMAT0004518

CCAAUAUUACUGUGCUGCUUUA (SEQ ID NO:34)


**hsa-miR-30a-3p**

miRBase accession number: MIMAT0000088

CUUUCAGUCGGAUGUUUGCAGC (SEQ ID NO:35)


**hsa-miR-146b-5p**

miRBase accession number: MIMAT0002809

UGAGAACUGAAUUCCAUAGGCU (SEQ ID NO:36)


**hsa-miR-195-5p**

miRBase accession number: MIMAT0000461

UAGCAGCACAGAAAUAUUGGC (SEQ ID NO:37)


**hsa-let-7f-2-3p**

miRBase accession number: MIMAT0004487

CUAUACAGUCUACUGUCUUUCC (SEQ ID NO:38)

FIGURE 8 (continued)

**hsa-miR-34a-3p**

miRBase accession number: MIMAT0004457

CAAUCAGCAAGUAUACUGCCCU (SEQ ID NO:39)

**hsa-miR-423-5p**

miRBase accession number: MIMAT0004748

UGAGGGGCAGAGAGCGAGACUUU (SEQ ID NO:40)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ROLLASON et al.** *Genotypic antimicrobial characterization of Propionibacterium acnes isolates from surgically excised lumbar disc herniations,* 2013
- **ALEXEYEV OA et al.** Direct visualization of Propionibacterium acnes in prostate tissue by multicolor fluorescent in situ hybridization assay. *J Clin Microbiol,* November 2007, vol. 45 (11), 3721-8
- **ACHERMANN Y et al.** Propionibacterium acnes: from commensal to opportunistic biofilm-associated implant pathogen. *Clin Microbiol Rev,* 2014, vol. 27, 419-40
- **LUPAN I et al.** The evidence of contaminant bacterial DNA in several commercial Tag polymerases. *Rom Biotech Lett,* 2013, vol. 18, 8007-12
- **R. SIMON.** Diagnostic and prognostic prediction using gene expression profiles in high-dimensional microarray data. *British Journal of Cancer,* 2003, vol. 89, 1599-1604